(19) Europäisches Patentamt
European Patent Office
Office européen des brevets

(11) EP 4 003 324 B1

(12) EUROPEAN PATENT SPECIFICATION

(45) Date of publication and mention of the grant of the patent:
**18.09.2024 Bulletin 2024/38**

(21) Application number: **20753432.2**

(22) Date of filing: **24.07.2020**

(51) International Patent Classification (IPC):
***A61K 31/215*** (2006.01) ***A61K 31/192*** (2006.01)
***A61K 31/225*** (2006.01) ***A61K 31/7024*** (2006.01)
***A61K 31/7028*** (2006.01) ***A61K 38/12*** (2006.01)
***A61K 45/06*** (2006.01) ***A61P 11/00*** (2006.01)
***A61P 31/04*** (2006.01) ***A61K 9/00*** (2006.01)
***A61L 15/44*** (2006.01) ***A61L 26/00*** (2006.01)
***C07C 13/32*** (2006.01) ***C07C 31/137*** (2006.01)
***C07C 69/36*** (2006.01) ***C07C 69/38*** (2006.01)
***C07C 69/40*** (2006.01) ***C07C 69/653*** (2006.01)
***C07C 69/657*** (2006.01) ***C07H 15/256*** (2006.01)

(52) Cooperative Patent Classification (CPC):
**A61K 31/215; A61K 31/7028; A61K 45/06; A61L 15/44; A61L 26/0066; A61P 11/00; A61P 31/04; C07C 31/137; C07C 69/36; C07C 69/38; C07C 69/40; C07C 69/653; C07C 69/657; C07H 15/256;** A61L 2300/404;

(Cont.)

(86) International application number:
**PCT/IB2020/057019**

(87) International publication number:
**WO 2021/014422 (28.01.2021 Gazette 2021/04)**

(54) **COMPOUNDS FOR USE AS ADJUVANTS IN ANTIBIOTIC THERAPY**

VERBINDUNGEN ZUR ANWENDUNG ALS ADJUVANS BEI DER ANTIBIOTIKATHERAPIE

COMPOSÉS POUR LEUR UTILISATION COMME ADJUVANTS DANS LA THÉRAPIE ANTIBIOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2019 IT 201900012888**

(43) Date of publication of application:
**01.06.2022 Bulletin 2022/22**

(73) Proprietors:
- **Università degli Studi di Roma "La Sapienza" 00185 Roma (IT)**
- **Fondazione Istituto Italiano di Tecnologia 16163 Genova (IT)**

(72) Inventors:
- **IMPERI, Francesco 00146 Roma RM (IT)**
- **ASCENZIONI, Fiorentina 00185 Roma RM (IT)**
- **MORI, Mattia 16163 Genova GE (IT)**
- **GHIRGA, Francesca 16163 Genova GE (IT)**
- **QUAGLIO, Deborah 00185 Roma RM (IT)**
- **CORRADI, Silvia 00185 Roma RM (IT)**
- **LO SCIUTO, Alessandra 00146 Roma RM (IT)**
- **BOTTA, Bruno 00185 Roma RM (IT)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **CALCATERRA, Andrea**
**00185 Roma RM (IT)**
• **STEFANELLI, Roberta**
**88100 Catanzaro CZ (IT)**

(74) Representative: **Santoro, Sofia et al**
**Società Italiana Brevetti S.p.A.**
**Via Giosuè Carducci, 8**
**20123 Milano (IT)**

(56) References cited:

- **DREWES S E ET AL: "Antimicrobial monomeric and dimeric diterpenes from the leaves of Helichrysum tenax var tenax", PHYTOCHEMISTRY,, vol. 67, no. 7, 1 April 2006 (2006-04-01), pages 716 - 722, XP028059720, ISSN: 0031-9422, [retrieved on 20060401], DOI: 10.1016/J.PHYTOCHEM.2005.12.015**
- **JAMES D MCCHESNEY ET AL: "Antimicrobial Diterpenes of Croton sonderianus. II. ent-Beyer-15-en-18-oic Acid", PHARMACUETICAL RESEARCH, vol. 8, no. 10, 1 October 1991 (1991-10-01), pages 1243 - 1247, XP055675324, DOI: https://doi.org/10.1023/A:1015891410300**
- **CANCAN CAI ET AL: "Two new kaurane-type diterpenoids from Wedelia chinensis (Osbeck.) Merr", NATURAL PRODUCT RESEARCH, vol. 31, no. 21, 2 November 2017 (2017-11-02), GB, pages 2531 - 2536, XP055675335, ISSN: 1478-6419, DOI: 10.1080/14786419.2017.1317775**
- **DEBORAH QUAGLIO ET AL: "Structural Elucidation and Antimicrobial Characterization of Novel Diterpenoids from Fabiana densa var. ramulosa", ACS MEDICINAL CHEMISTRY LETTERS, vol. 11, no. 3, 30 January 2020 (2020-01-30), US, XP055675356, ISSN: 1948-5875, DOI: 10.1021/acsmedchemlett.9b00605**

(52) Cooperative Patent Classification (CPC): (Cont.)
A61L 2300/406; C07C 2603/86

## Description

BACKGROUD ART

Antibiotic resistance represents one of the greatest threats to human health as it undermines the effectiveness of the compounds available for the treatment of the most common bacterial infections. In particular, the increase in antibiotic resistance in Gram-negative bacteria such as *Pseudomonas aeruginosa, Acinetobacter baumannii* and *Enterobacteriaceae,* is considered by the World Health Organization (WHO) to be a priority problem for which new antibiotics are urgently needed (https://www.who.int/en/news-room/detail/27-02-2017-who-publishes-list-of-bacteria-for-which-new-antibiotics-are-urgently-needed). Furthermore, the diffusion of antibiotic resistances is accompanied by a drastic reduction in the discovery of new antibiotics (Payne et al., 2007).

The polymyxins, such as colistin (polymyxin E) and polymyxin B, are increasingly used as last-line therapeutic options for the treatment of infections caused by multiresistant Gram-negative bacteria (Giamarellou, 2016; Poirel et al., 2017). The polymyxins consist of a cyclic heptapeptide ring and by a lateral tripeptide chain acylated to the N-terminal with a fatty acid tail. Polymyxins target the outer membrane of the Gram-negative bacteria with which they electrostatically interact. Electrostatic interaction occurs between amino groups of colistin and negatively charged groups present in lipid A of lipopolysaccharide (LPS), responsible for anchoring LPS to the external membrane of Gram-negative bacteria. Although the mechanism of action of colistin is not yet fully known, the colistin-lipid A interaction is believed to destabilize the outer membrane resulting in increased membrane permeability and bacterium death (Poirel et al., 2017).

However, with the use of colistin, colistin-resistant bacteria have emerged (Poirel et al., 2017). At present, colistin-resistance in *Klebsiella pneumoniae, P. aeruginosa* and *A. baumannii* appears to be limited. However, much higher resistance rates of up to 50% have been reported in the literature (Giamarellou, 2016). Furthermore, epidemiological studies of colistin resistance can provide an underestimation of the real extent of the phenomenon as to date there are no automated methods for detecting colistin resistance in the clinical setting (Jayol et al., 2018).

Colistin-resistance develops through the activation of lipid A modification systems which, once modified, is unable to interact with the antibiotic. The mechanisms responsible for these modifications include the addition of 4-amino-4-deoxy-L-arabinose (Ara4N) or phosphoethanolamine (PEtN) to lipid A. Both of these modifications reduce the net negative charge of the LPS and, therefore, the affinity of this for colistin, making the bacterium insensitive and therefore resistant to the antibiotic (Poirel et al., 2017).

More specifically, epidemiological studies and experimental evidence suggest that the prevailing molecular mechanism that confers colistin resistance in various gram-negative bacteria, including P. *aeruginosa,* is the enzymatic transfer of Ara4N to lipid A (Nowicki et al., 2015; Pedersen et al., 2017; Lo Sciuto e Imperi, 2018). This modification occurs through a complex series of reactions wherein the last passage is catalysed by the enzyme ArnT, an Ara4N transferase located on the cytoplasmic membrane (Petrou et al., 2016). These data suggest that ArnT inhibition may block colistin resistance in those bacteria that possess this resistance mechanism, just to name a few, *P. aeruginosa, Klebsiella pneumoniae, Salmonella typhimurium, Escherichia coli* (see, for example, Protein Expression and Purification, Volume 46, Issue 1, March 2006, Pages 33-39; "Purification and characterization of the L-Ara4N transferase protein ArnT from Salmonella typhimurium", Lynn E. Bretscher et al. https://doi.org/ 10.1016/j.pep.2005.08.028), *Burkholderia cenocepacia, Yersinia pestis, Yersinia enterocolitica, Proteus mirabilis and Salmonella enterica serovar Typhimurium* (see, for example, ChemBioChem 2019, 20,2936-2948; "Synthetic Phosphodiester-Linked 4-Amino-4-deoxy-l-arabinose Derivatives Demonstrate that ArnT is an Inverting Aminoarabinosyl Transferase", Charlotte Olagnon et al., DOI: 10.1002/cbic.201900349). Recently, the crystalline structure of ArnT has been resolved and the atomic details of its binding pocket for Ara4N have been clarified, making it possible to use *in silico* selection systems to identify inhibitors of ArnT, a very promising target for the development of inhibitors of colistin resistance mediated by this molecular mechanism (Petrou et al., 2016).

Potential inhibitors of colistin resistance mediated by lipid A aminoarabinosylation have been described in the literature. For example, Kline T, et al. (Synthesis of and evaluation of lipid A modification by 4-substituted 4-deoxy arabinose analogues as potential inhibitors of bacterial polymyxin resistance. Bioorg Med Chem Lett. 2008) describes an aminoarabinose analogue capable of causing a slight decrease in the degree of lipid A aminorabinosylation in an *in vitro* assay on membranes purified from the bacterium *Salmonella typhimurium.* However, said analogue has not been able to inhibit polymyxins resistance at any tested concentration (up to 90 mM).

Moreover, the international patent application WO2017083859 describes some potential inhibitors of the ArnT enzyme identified by *in silico* screening. These potential inhibitors were tested for the ability to inhibit the growth of *Escherichia coli* BL21 which expressed its ArnT enzyme or that of S. *typhimurium,* in the presence or absence of polymyxin B (a polymyxin with an activity equivalent to that of colistin). None of the identified compounds was able to completely inhibit the growth of the "test" strains and many of them showed a high growth inhibition activity even in the absence of antibiotic (Figures 8, 10, 12 and 14), suggesting that these compounds may have non-specific antibacterial activity ("off-target activity"), independent of their possible ability to inhibit the ArnT enzyme.

It is also known in the literature that some diterpene or diterpenoic derivatives, in particular derivatives of beyerenic or kauranic acid, have a certain antibacterial activity mainly against Gram-positive bacteria, such as *Staphylococcus aureus* (see, for example, Cai C. et al., "Two new kaurane-type diterpenoids from Wedelia chinensis (Osbeck.) Merr", Nat. Prod. Res. 2017, 31(21), 25-31), S. *aureus, Enterococcus faecalis, Bacillus subtilis* and *Staphylococcus epidermidis* (see, for example, Drewes S. E. et al., "Antimicrobial monomeric and dimeric diterpenes from the leaves of Helichrysum tenax var tenax", Phytochem. 2006, 67(7), 716), as well as against *6 subtilis, S. aureus* and *Mycobacterium smegmatis* (see, for example, "Antimicrobial diterpenes of Croton sondenarius. II ent-Beyer-15-en-18-oic Acid", McChesney J.D. et al., Pharm. Res. 1991, 8(10), 1243). However, none of the identified compounds is presented as useful in the treatment of antibiotic-resistant bacterial infections, in particular polymyxin-resistant, nor as a possible inhibitor of antibiotic resistance or adjuvant of antibiotics.

Therefore, the need is still felt for alternative therapeutic solutions able of effectively treating antibiotic-resistant bacterial infections and, more particularly, of increasing the efficacy and clinical duration of polymyxins, such as for example polymyxin E (colistin) and B, preventing the development of resistance to such antibiotics and/or of restoring sensitivity to said antibiotics in already resistant strains.

SUMMARY OF THE INVENTION

By *in silico* screening of a vast library of natural compounds with respect to the crystallographic structure of the ArnT protein in complex with the undecaprenyl phosphate ligand, the inventors have now found that ent-beyer-15-en-18-O-oxalic acid (BBN149), a natural diterpene isolated from the leaves of the *Fabiana densa var. ramulosa,* and the natural or synthetic derivatives thereof are valid inhibitors/antagonists of ArnT, the enzyme responsible for the aminoribosilation of lipid A of LPS, one of the main mechanisms of antibiotic resistance in bacteria.

Therefore, the invention refers to compounds of formula (I):

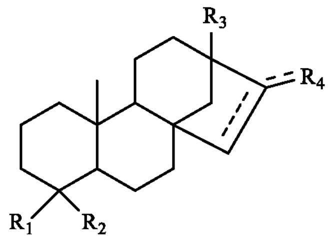

I

wherein

the compound is selected from glucopyranosyl β-D ester of ent-beyeran-19-oic acid (**SR4**), ent-beyer-15-en-18-O-malonic acid (**FDM**) and ent-beyeran-18-O-oxalic acid (**FDO-H**) as claimed in claim 3 and to the use thereof as a medicament as claimed in claim 4.

The invention also relates to compounds of formula (I) wherein the compound is selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**) and ent-beyeran-18-O-oxalic acid (**FDO**-**H**) and pharmaceutically acceptable salts thereof for use as an adjuvant of an antibiotic therapy in the treatment of polymyxins-resistant bacterial infections as claimed in claim 1.

Furthermore, the invention relates to combinations of one or more of the compounds of formula (I) as defined above with at least one other active principle, in particular an antibacterial and/or antibiotic agent as claimed in claim 8, and to compositions comprising one or more compounds of formula (I) as defined above or the combination according to the present invention and at least one pharmaceutically acceptable excipient and/or carrier as claimed in claim 10.

The invention also relates to products, in particular medical devices, comprising at least one compound, a combination or a composition according to the present invention as claimed in claim 12.

Moreover, the invention relates to the *in vitro* use of compounds of formula (I) as defined above to sensitize a bacterium to an antibacterial agent or an antibiotic, for example, to an antibiotic belonging to the class of polymyxins such as colistin (polymyxin E) or polymyxin B as claimed in claim 13.

The invention also relates to an *in vitro* method for sensitizing a bacterium to an antibacterial agent or an antibiotic which comprises the exposure of said bacterium to one or more compounds of formula (I) as claimed in claim 14. In particular, the *in vitro* method for sensitizing a bacterium to an antibacterial agent or an antibiotic of the invention can comprise the exposure of said bacterium to one or more compounds of formula (I) together with colistin or even to the combinations, compositions or products as defined above.

## DETAILED DESCRIPTION OF FIGURES

Figure 1. Checkerboard assays to evaluate the synergistic activity between the compounds of interest, here in particular BBN149, FDS, FDM, SR4, SR8 and SR10, and colistin against the colistin-resistant strain *P. aeruginosa* PA14 $col^R$ 5. The grey boxes highlight the combinations colistin/compound of interest capable of causing a growth inhibition of at least 90%.

Figure 2. Graphs showing the adjuvant effect (grey line) of the compounds of interest, here in particular BBN149, FDM, FDS, SR4, SR8, SR10 and FDO-H, in increasing concentration (abscissa axis) on the MIC of colistin (ordinate axis) against the colistin-resistant strain P. *aeruginosa* PA14 $col^R$ 5. The adjuvant effect was determined by checkerboard assay. As a control, DMSO at equivalent concentrations (0.02-0.64%; black line) was used. The data shown in the graphs are representative of at least three independent experiments.

## GLOSSARY

In the context of the present description, the term "*effective amount*" means amount of active compound, association or composition comprising the active compounds of the invention, sufficiently high to provide the desired benefits and at the same time low enough not to cause serious side effects.

In the context of the present description, "*about*" refers to the experimental error that may occur during conventional measurements. More specifically, when referring to a value it indicates $\pm$ 5% of the indicated value and when referring to a range $\pm$ 5% of the extremes thereof.

In the context of the present description, the terms "*synergism*", "*synergy*", "*synergistic activity*", "*activity in synergy with*" and the like must be read in their broadest meaning of simultaneous action of two or more compounds which is generally expressed in a positive sense, that is, in the enhancement of the effectiveness of one or both of them. In particular, the above terms also mean "adjuvant", "adjuvant activity" and "adjuvant activity with", or "co-adjuvant", respectively.

In the context of the present description, with the wording "*antibiotic resistance mediated by the ArnT enzyme*" is meant the phenomenon for which a bacterium is resistant to an antimicrobial drug, for example colistin, due to the action of the ArnT enzyme, the enzyme responsible for the aminoaribosylation of the lipid A component of the lipopolysaccharide (LPS). The term "*mediated by*" is therefore interchangeable with the terms initiated by, promoted by, caused by, exacerbated by and the like. The antibiotic resistance mediated by the ArnT enzyme can be easily identified, for example, by mass spectrometry analysis as described in greater detail in the following detailed description.

## DETAILED DESCRIPTION OF THE INVENTION

The authors of the present invention have isolated and selected diterpenes of natural origin and developed synthetic derivatives thereof capable of inhibiting the ArnT, the enzyme responsible for the aminoaribosylation of the lipid A component of the lipopolysaccharide (LPS).

In detail, a library consisting of over 1000 compounds of natural origin extracted, purified and characterized by different plants of traditional medicine and synthetic derivatives thereof was screened *in silico* with respect to the crystallographic structure of the ArnT enzyme in complex with the ligand undecaprenyl phosphate to identify new compounds capable of contrasting/inhibiting the activity of the ArnT enzyme and therefore enhancing the effectiveness of antibiotics in the treatment of bacterial infections caused by antibiotic-resistant Gram-negative bacteria.

By *in silico* screening, 18 compounds were identified (see, Table 1) which were then tested for their antibacterial activity and for their synergistic or adjuvant activity with different antibiotics, in particular colistin, against an antibiotic-resistant strain, in particular colistin-resistant, of *P. aeruginosa* grown in the absence or presence of a sub-inhibitory concentration of colistin.

The results obtained from the microbiological essays indicated the compound ent-beyer-15-en-18-O-oxalic acid (BNN149) and its derivatives as new potential agents capable of increasing the effectiveness of antibiotics in the treatment of antibiotic-resistant bacterial infections and, in particular, resistant to colistin.

Therefore, the present invention generally discloses compounds of formula (I):

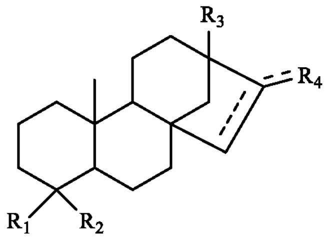

I

wherein

> **$R_1$** and **$R_2$** are the same or different and independently selected among: hydrogen, $C(R_A)_3$, $OR_A$, $C(=O)R_A$, $C(=O)OR_A$, $CH_2OR_A$, $CH_2OC(=O)R_A$, $CH_2OC(=O)OR_A$, $CH_2OC(=O)(CH_2)_nC(=O)OR_A$ with n = 0, 1 or 2, $CH_2N(R_A)_2$, $C(=O)N(R_A)_2$, $CH_2NHC(=O)R_A$ and $CH_2C(=O)N(R_A)_2$; where **$R_A$** is selected among: hydrogen, alkyl, hydroxyl, monosaccharide, disaccharide and $CH_2$-formula (I);
> **$R_3$** is hydrogen, $C(R_B)_3$ or $OR_B$; where **$R_B$** is selected among hydrogen, alkyl, hydroxyl and disaccharide;
> the endocyclic symbol - - - represents a single or double bond and, when it represents a double bond, the exocyclic symbol - - - binding $R_4$ to the carbocycle represents a single bond;
> **$R_4$** is hydrogen when the exocyclic symbol - - - binding $R_4$ to the carbocycle represents a single bond; or **$R_4$** is oxygen or methylene when the exocyclic symbol - - - binding $R_4$ to the carbocycle represents a double bond; and pharmaceutically acceptable salts thereof for the use as an adjuvant in an antibiotic therapy in the treatment of polymyxins-resistant bacterial infections.

More in particular, the present invention discloses compounds of formula (I) wherein **$R_1$** is selected among: $C(=O)R_A$, $C(=O)OR_A$, $CH_2OR_A$, $CH_2OC(=O)R_A$, $CH_2OC(=O)(CH_2)_nC(=O)OR_A$ with n = 0, 1 or 2, wherein **$R_A$** is selected among: hydrogen, methyl, hydroxyl, monosaccharide and $CH_2$-formula I;
**$R_2$** is methyl:

> **$R_3$ is** selected among: hydrogen, methyl, hydroxyl and disaccharide;
> the endocyclic symbol - - - represents a single or double bond and, when represents a double bond, the exocyclic symbol - - - binding $R_4$ to the carbocycle represents a single bond;
> **$R_4$** is hydrogen when the exocyclic symbol - - - binding $R_4$ to the carbocycle represents a single bond; or **$R_4$** is oxygen or methylene when the exocyclic bond - - - binding $R_4$ to the carbocycle represents a double bond; and pharmaceutically acceptable salts thereof for the use as an adjuvant in an antibiotic therapy, particularly for the use in the treatment of bacterial infections, even more particularly antibiotic-resistant bacterial infections.

$R_1$ can be selected among: $CH_2OH$, $C(=O)OH$, $C(=O)OCH_3$, $CH_2OC(=O)(CH_2)_2C(=O)OH$, $CH_2OC(=O)C(=O)OH$, $CH_2OC(=O)CH_2C(=O)OH$, C(=O)O-monosaccharide and $CH_2OC(=O)(CH_2)_nC(=O)O$-$CH_2$-formula (I) with n = 0, 1 or 2.

The terms monosaccharide and disaccharide in the context of the present invention are as commonly understood in the art and can thus indicate, respectively, any monosaccharide (for example glucose, fructose, galactose and mannose) and any disaccharide (for example sucrose, maltose, lactose, trehalose, gentiobiose and cellobiose). The monosaccharide is selected between glucose and o-acetyl-glucose or the disaccharide is selected among sucrose, maltose and lactose.

In particular, the invention discloses a compound for the use as an adjuvant in an antibiotic therapy, wherein the compound is selected among: ent-beyer-15-en-18-ol (**FDA**); ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl ester of 4-α-13-[(2-O-β-D-glucopyranosyl-β-D-glucopyranosyl) oxy]-16β-hydroxy-entkaur-16-en-19-oic] acid (**SR1**); ent-16-oxo-beyeran-19-oic acid (**SR2**); 1,3,4,6-Tetra-O-acetyl-β-D-glucopyranosyl ester of ent-beyeran-19-oic acid (**SR3**); glucopyranosyl β-D ester of ent-beyeran-19-oic acid (**SR4**); 13-hydroxy-kaur-16-en-19-oic acid (**SR5**); 4-α-13-[(2-O-β-D-glucopyranosyl-β-D glucopyranosyl) kaur-16-en-19-oic) acid (**SR6**); methyl ester of ent-16-oxo-beyeran-19-oic acid (**SR7**); ent-beyeran-19-O-oxalic acid (**SR8**); ent-beyeran-19-ol (**SR9**); ent-beyeran-19-oic acid (**SR10**) and ent-beyeran-18-O-oxalic acid (**FDO-H**).

Even more in particular, the invention refers to a compound selected among: ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**) and ent-beyeran-18-O-oxalic acid (**FDO-H**) and pharmaceutically acceptable salts thereof for the use as an adjuvant in antibiotic

therapy in the treatment of polymyxins-resistant bacterial infections as claimed in claim 1.

The bacterial infections mentioned above can be, for example, bacterial infections caused by Gram-negative bacteria such as *Pseudomonas aeruginosa, Escherichia coli, Klebsiella pneumoniae, Klebsiella oxytoca, Enterobacter spp, Citrobacter freundii or Acinetobacter baumanni.* More in particular, infections caused by *Pseudomonas aeruginosa.*

The antibiotic-resistant bacterial infections mentioned above can be, for example, antibiotic-resistant bacterial infections wherein the antibiotic-resistance is mediated by the enzyme transferase ArnT.

The antibiotic resistance mediated by ArnT, in particular the resistance to colistin mediated (caused, promoted, initiated, exacerbated) by ArnT, can be detected or determined according to any one of the methods known in the art, for example, using the analysis of lipid A by mass spectrometry, as for example described in (Lo Sciuto A, Martorana AM, Fernández-Piñar R, Mancone C, Polissi A, Imperi F. Pseudomonas aeruginosa LptE is crucial for LptD assembly, cell envelope integrity, antibiotic resistance and virulence. Virulence. 2018;9(1): 1718-1733. doi:10.1080/21505594.2018.1537730).

By way of example, but in no limitative way, the analysis of mass spectrometry to determine the modification of lipid A with the aminoarabinose, catalysed by ArnT, can be carried out according to the following experimental protocol. For the extraction of lipid A, 2 mL of a bacterial culture in an early stationary phase are centrifuged at 3000 $\times$ g for 10 minutes and the bacterial sediment resuspended in 400 $\mu$L of 70% isobutyric acid and 1M ammonium hydroxide (in a 5:3 ratio). The samples are incubated for 1 hour at 100°C and centrifuged at 2000 $\times$ g for 15 minutes. The supernatants are added to 400 $\mu$L of water free of endotoxins, frozen at -80°C and freeze-dried in a vacuum centrifuge. The resulting sediment is washed with 1 mL of methanol and lipid A is extracted from the sediment using 100 $\mu$L of a solution of chloroform, methanol and water (in a 3:1:0.25 ratio). After centrifugation at 2000 $\times$ g for 15 minutes, 2 $\mu$L of supernatant are mixed with 2 $\mu$L of norharmane matrix resuspended at 10 mg/ml in a solution of chloroform and methanol (in a 2:1 ratio) and 0.5 $\mu$L of this mixture are analysed in a time-of-flight mass spectrometer with laser desorption/ionisation assisted by matrix (Matrix-assisted laser desorption/ionization Time of flight or MALDI-TOF) (5800 MALDI TOF/TOF Analyzer, Sciex). The spectral data can be analyzed with the 4000 series Explorer software version 4.1.0 (Sciex, Ontario, Canada) and used to estimate the lipid A forms based on the structures and molecular weights predicted on the basis of the literature for each bacterium. A reference strain, which does not present aminoarbinose on lipid A, may be included in the analysis as a comparison.

Therefore, the present invention also discloses compounds according to any one of the embodiment herein described for the use in the treatment of bacterial infections, more particularly of antibiotic-resistant bacterial infections, still more particularly antibiotic-resistant bacterial infections wherein the antibiotic-resistance is mediated by Arnt, wherein the antibiotic resistance mediated by Arnt is determined, for example, by extraction of the lipid A and mass spectrometry.

The above-mentioned antibiotic-resistant bacterial infections can be, for example, polymyxins-resistant bacterial infections, in particular colistin- or polymyxin B-resistant, wherein the antibiotic-resistance is mediated by the enzyme transferase ArnT.

The antibiotic resistance mediated by ArnT, in particular the resistance to colistin mediated (caused, promoted, initiated, exacerbated) by ArnT, can be identified or determined according to any of the methods known in the art, for example, using the method of microdilutions (BMD, Broth microdilution) for the determination of the minimum inhibitory concentration (MIC), test approved by the European Committee on Antibiotic Susceptibility Testing (EUCAST) and by the Clinical and Laboratory Standards Institute (CLSI) (see, European Committee on Antimicrobial Susceptibility Testing (EUCAST), Breakpoint Tables for Interpretation of MICs and Zone Diameters, Vol. 8, EUCAST, Va¨xjo¨, Sweden, 2018; and CLSI, Clinical and Laboratory Standards Institute, M07-A10: Methods for Dilution Antimicrobial Susceptibility Tests for Bacteria That Grow Aerobically; Approved Standard, Vol. 35, CLSI, Wayne, PA, USA, 10th edition, 2015). By way of example, MIC assays can be performed in 96-well microtitration plates. In each column of wells are aliquoted 100 $\mu$l of MH medium containing decreasing concentrations of colistin (dilutions in reason of 2 from 256 to 0.5 $\mu$g/ml) or devoid of colistin, and in each row of wells further 100 $\mu$l of MH medium containing $1\times10^6$ cells/ml of each bacterial strain of interest, so as to reach a final volume of 200 $\mu$l, a concentration of bacteria equal to $0.5\times10^5$ cells/ml and colistin concentrations in the range 0-128 $\mu$g/ml. The plates are incubated at 37°C without stirring for 24 hours, and the MIC is visually assessed as the minimum concentration of colistin capable of causing absence of turbidity in the well.

Therefore, the present invention also discloses compounds according to any embodiment herein described for the use in the treatment of bacterial infections, in particular antibiotic-resistant bacterial infections, more particularly polymyxin-resistant bacterial infections, even more particularly colistin-resistant, wherein the resistance to colistin is determined by the microdilution method.

In addition to those mentioned above, infections known in the art for possessing this antibiotic-resistance mechanism, in particular to colistin, are those caused for example by *Salmonella typhimurium, Burkholderia cenocepacia, Yersinia pestis* and *Yersinia enterocolitica, Proteus mirabilis* and *Salmonella enterica serovar Typhimurium.*

In a preferred embodiment, the bacterial infection may be an acute or chronic pulmonary, extra-pulmonary localized or systemic infection.

The compounds of the invention can be advantageously associated in a combination product comprising one

or more compounds of formula (I) (*i.e.* an ArnT inhibitor) and at least one other active principle.

Therefore, the present invention also refers to the combination as claimed in claim 8 of one or more of the compounds as defined above with at least one other active principle. In one embodiment, said active principle is an antibacterial agent and/or an antibiotic.

The antibacterial agent and/or the antibiotic can be any antibacterial agent or antibiotic, in particular any antibacterial agent or antibiotic the mechanism of action of which provides for electrostatic interaction with the lipopolysaccharide of the bacterial wall. In one embodiment, the antibiotic can be a peptididic antibiotic. In a preferred embodiment, the antibiotic belongs to the class of polymyxins and is preferably colistin (polymyxin E) or polymyxin B, more preferably colistin. The efficacy of polymyxins, in particular colistin and polymyxin B, can be undermined by bacteria that have the same resistance mechanism, that is the one mediated by ArnT (see Jeannot K, Bolard A, Plésiat P. Resistance to polymyxins in Gram-negative organisms. Int J Antimicrob Agents. 2017; 49(5): 526-535. Doi: 10.1016/j.ijantimicag.2016.11.029).

Although this is not an essential feature for the purposes of therapeutic efficacy, the inventors of the present invention have also found that, when the weight ratio between compound of formula (I) and antibiotic in the association of the present invention is between 1:1-1:20, the association is able to perform its beneficial effects optimally.

The compounds and the combination of the invention can be included in a pharmaceutical composition.

Therefore, the present invention also relates to compositions as claimed in claim 10 comprising, or consisting of, one or more compounds of the invention and at least one suitable pharmaceutically acceptable excipient or carrier, or to compositions comprising the combination of the invention and at least one suitable pharmaceutically acceptable excipient or carrier. In one embodiment, the invention relates to compositions comprising one or more compounds of formula (I), at least one antibacterial agent and/or an antibiotic and at least one suitable pharmaceutically acceptable excipient or carrier.

Said excipient and/or additive may be selected among those generally known in the art such as, for example: carriers, fillers, humectants, disintegrating agents, binders, retardants, absorption accelerators, wetting agents, surfactants, adsorbents, lubricants, glidants, flavouring agents, sweeteners and/or preservatives.

The skilled in the art will be able to select appropriate additives/excipients thanks to the general knowledge in the field.

The compositions according to any of the embodiments provided in the present description, can be formulated in any form, administered by any route of administration and associated with any other component, in a variety of ways.

In particular, the compositions of the invention can be in liquid, semi-liquid, solid or semi-solid form.

The compositions are preferably, but not exclusively, administered orally or topically. Suitable liquid forms are, by way of non-limiting example, drops, emulsions, solutions, suspensions (prepared or extemporaneous), syrups and elixirs. The liquid or semi-liquid formulations may be contained in suitable delivery carriers. Suitable solid forms are, by way of non-limiting example, tablets, hard or soft capsules, pills, jellies, lozenges, powders, granulates, sachets and films. The solid dosage forms may also be coated with enteric, gastric or other coatings known in the state of the art. Suitable semi-solid forms are, by way of non-limiting example, ointments, gels, salves, creams and pastes.

In a preferred embodiment, the composition is formulated in the form of a solution, suspension, cream or ointment.

The formulations, according to any of the embodiments herein described, can be prepared according to conventional methods known to the person skilled in the art.

Moreover, the compounds, the combinations or the compositions of the invention as defined above may be included in a suitable medical device useful in the treatment of bacterial infections. Non-limiting examples of medical devices suitable for the purposes of the present invention are: bandages, gauzes, patches, cotton wool, spray, prostheses, probes *etc.*

Therefore, the present invention also relates to medical devices comprising one or more compounds, the combination or composition of the invention, wherein the compounds, the combination and composition are as defined above. In particular, the invention relates to bandages, gauzes, patches, cotton wool, sprays, prostheses, probes, as claimed in claim 12 preferably bandages, gauzes and patches, comprising one or more compounds, the combination or composition of the invention, where the compounds, the combination and composition are as defined above.

The different components or active ingredients of the composition of the invention can be present in variable quantities.

Furthermore, according to any of the embodiments, the compounds, the combination, the compositions and/or the medical devices of the present invention are mainly intended for use by humans, but can also be used on animals.

The present invention also relates to the *in vitro* use as claimed in claim 13 of the compounds, combination or compositions as described above, for sensitizing a bacterium, in particular a gram-negative bacterium as defined above, to an antibacterial agent or an antibiotic. In particular, the antibiotic can be an antibiotic belonging to the class of polymyxins such as, for example, colistin (polymyxin E) or polymyxin B.

The present invention therefore also refers to an *in vitro* method as claimed in claim 14 for sensitizing a bacterium

to an antibacterial agent or an antibiotic which comprises the exposure of a bacterium to one or more compounds of formula (I) or to the combination of the invention.

As anticipated above, in addition to the compounds of formula (I), the inventors have also isolated novel diterpenes of natural origin and designed and developed several synthetic analogues with ent-beyeranic and ent-kauranic structure of BNN149 so as to enhance the activity and selectivity thereof towards the ArnT enzyme.

Therefore, the present invention also discloses compounds of formula (I'):

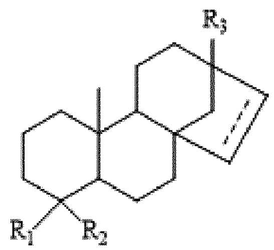

(I')

wherein

- **$R_1$** is selected among $C(=O)OR_A$, $CH_2OR_A$ and $CH_2OC(=O)CH_2C(=O)OR_A$, where **$R_A$** is selected among hydrogen and monosaccharide (where monosaccharide is as defined above);
- **$R_2$** and **$R_3$** are methyl; and
- the symbol - - - represents a single or double bond; provided that when - - - represents a single bond $R_1$ is not C(=O)OH and when - - - represents a double bond $R_1$ is not $-CH_2OH$; and pharmaceutically acceptable salts thereof.

In an embodiment, the present invention refers to a compound of formula (I') as defined above wherein

- **$R_1$** is selected among $C(=O)OR_A$, $CH_2OR_A$ and $CH_2OC(=O)CH_2C(=O)OR_A$, where **$R_A$** is selected among hydrogen and monosaccharide (where monosaccharide is as defined above);
- **$R_2$** and **$R_3$** are methyl; and
- the symbol - - - represents a single or double bond; provided that when - - - represents a single bond $R_1$ is not C(=O)OH and when - - - represents a double bond $R_1$ is not $-CH_2OH$ or - C(=O)OH; and pharmaceutically acceptable salts thereof.

The present invention discloses compounds of formula (I') wherein $R_1$ is selected among: $CH_2OH$, C(=O)OH, $C(=O)OCH_3$, $CH_2OC(=O)(CH_2)_2C(=O)OH$, $CH_2OC(=O)C(=O)OH$, $CH_2OC(=O)CH_2C(=O)OH$, C(=O)O-monosaccharide.

In particular, the present invention refers to a compound selected among: glucopyranosyl β-D ester of ent-beyeran-19-oic acid (**SR4**); ent-beyer-15-en-18-O-malonic acid (**FDM**) and ent-beyeran-18-O-oxalic acid (**FDO-H**) as claimed in claim 3.

The present invention also relates to the compounds **SR4, FDM** and **FDO-H** for use as a medicament. In particular, the invention also relates to their use as an adjuvant in an antibiotic therapy.

The present invention also relates to the compounds described above for the use in the treatment of bacterial infections, more particularly of antibiotic-resistant bacterial infections.

Similarly to what has been described above, the invention also refers to combinations, compositions and products comprising **SR4, FDM, FDO-H,** to their *in vitro* use in sensitizing antibiotic-resistant bacteria and to *in vitro* methods for sensitizing antibiotic-resistant bacteria to antibiotics comprising the exposure of antibiotic-resistant bacteria to said compounds, associations, compositions and/or products.

In addition, the present invention also discloses processes for the preparation of compounds of general formula (I') as defined above. More specifically, the invention discloses processes for the preparation of the compounds indicated as SR2, SR3, SR4, SR5, SR6, SR7, SR8, SR9, SR10 and FDO-H, as taught in the following examples and illustrated in the reaction schemes (Schemes 1-11).

All the compounds of the invention have been tested *in vitro* on the colistin-resistant reference strain of *P. aeruginosa* (PA14 $col^R$ 5).

The analysis of the results obtained by the microbiological essays showed that these compounds have a good ability to reduce the resistance to colistin on the PA14 $col^R$ 5 strain. Moreover, the selected or synthesized compounds

showed activity also towards other antibiotic-resistant strains of *P. aeruginosa* and for antibiotic-resistant clinical strains of other bacteria such as, for example, *Klebsiella pneumoniae.*

These results demonstrate that the compounds of the invention are active towards different bacterial species wherein the resistance mechanism is mediated by ArnT, just to name a few, *Pseudomonas aeruginosa, Escherichia coli, Klebsiella pneumoniae, Klebsiella oxytoca, Enterobacter spp, Citrobacter freundii, Salmonella typhimurium, Burkholderia cenocepacia, Yersinia pestis* and *Yersinia enterocolitica, Proteus mirabilis* and *Salmonella enterica serovar Typhimurium.* As specified above, the antibiotic resistance mediated by ArnT can be identified or determined according to any of the methods known in the art, for example, by using the method of extraction and analysis of lipid A by mass spectrometry as herein described.

Therefore, as will become clear from the following description and from the annexed examples, said compounds represent a valid alternative for the treatment of antibiotic-resistant bacterial infections, in particular when the resistance is mediated by the ArnT enzyme.

Other advantages of the compounds of the present invention will be immediately evident to the person skilled in the art on the basis of the previous description and of the examples reported below.

EXAM PLES

The examples reported below are for illustrative purposes only and are not intended to limit the scope of the present invention, which is defined in the appended claims.

*In silico screening*

The crystallographic structure of the ArnT protein in complex with the undecaprenyl phosphate ligand identified by the access code PDB 5F15 (Petrou VI, et al., 2016) was used as a rigid receptor in a virtual screening of a library of natural compounds consisting of about 1000 molecules, mainly extracted, isolated and characterized by plants used in traditional medicine of South America. The library is characterized by good chemical diversity and by compounds with drug-like characteristics (Lipinski et al., 1997). The visual inspection of the docking poses and the analysis of the relative scores allowed to select a restricted number of molecules that have been tested *in vitro* (Table 1).

Table 1. Structure and synergistic or adjuvant activity of the library compounds with a sub-inhibitory concentration of colistin (8 μg/ml).[1] The data were obtained on the *P. aeruginosa* PA14 Col$^R$5 strain, which has a value of MIC and $IC_{90}$ of colistin of 64 μg/ml.

| Compound | Structure | Without colistin $IC_{50}$ (μM) | Synergy with colistin (8 μg/ml) $IC_{50}$ (μM) | Synergy with colistin (8 μg/ml) $IC_{90}$ (μM) |
|---|---|---|---|---|
| BBN36 | 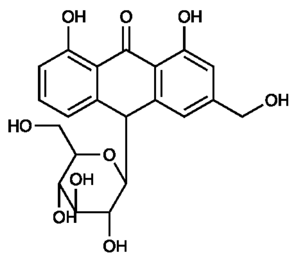 | >250 | >250 | >250 |
| BBN53 | 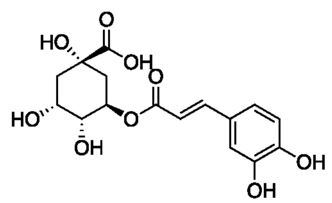 | >250 | >250 | >250 |

(continued)

| Compound | Structure | Without colistin $IC_{50}$ (μM) | Synergy with colistin (8 μg/ml) | |
|---|---|---|---|---|
| | | | $IC_{50}$ (μM) | $IC_{90}$ (μM) |
| BBN79 | 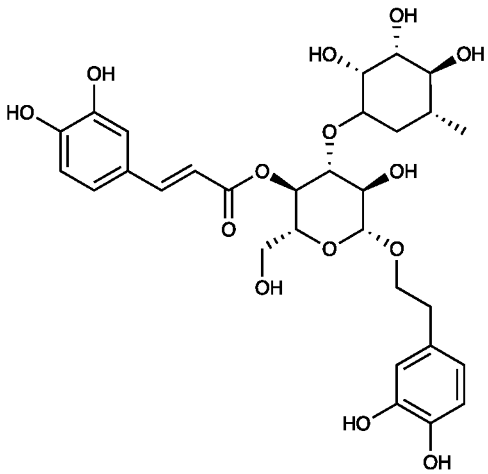 | >250 | 250 | >250 |
| BBN101 | 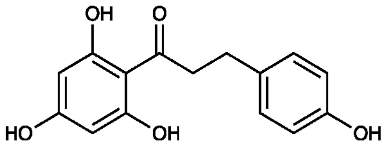 | >250 | >250 | >250 |
| BBN118 | 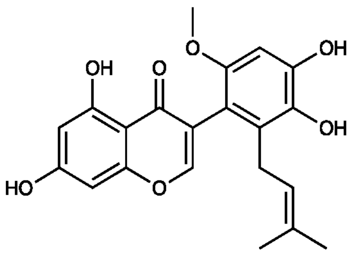 | >250 | 250 | >250 |
| BBN119 | 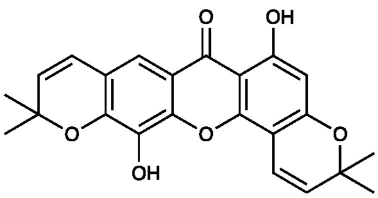 | >250 | >250 | >250 |
| BBN120 | 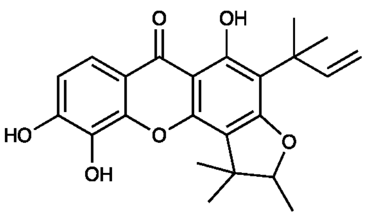 | >250 | 62.5 | >250 |
| BBN135 | 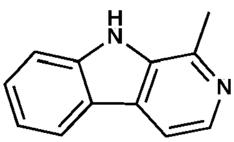 | >250 | >250 | >250 |

(continued)

| Compound | Structure | Without colistin $IC_{50}$ (µM) | Synergy with colistin (8 µg/ml) | |
|---|---|---|---|---|
| | | | $IC_{50}$ (µM) | $IC_{90}$ (µM) |
| BBN139 | 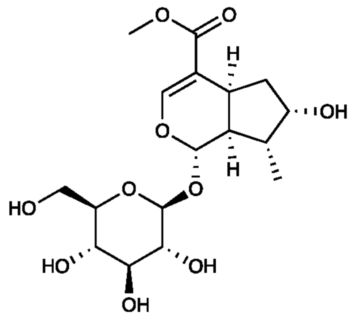 | >250 | >250 | >250 |
| BBN145 | 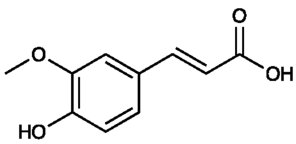 | >250 | >250 | >250 |
| BBN146 | 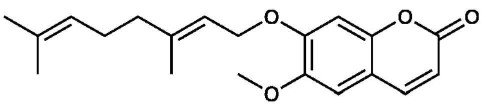 | >250 | >250 | >250 |
| BBN147 | 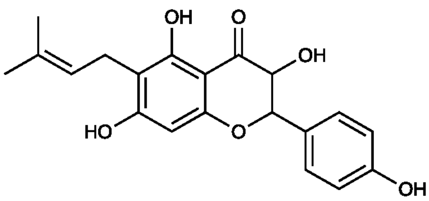 | >250 | 125 | >250 |
| BBN148 | 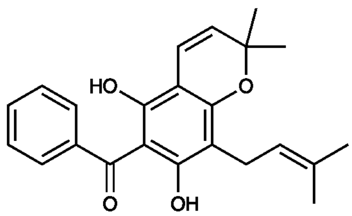 | >250 | >250 | >250 |
| BBN149 | 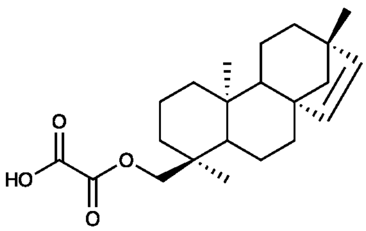 | >250 | 15.625 | 31.25 |
| BBN151 | 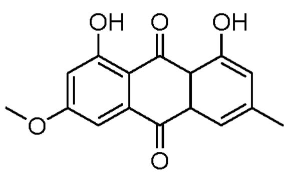 | >250 | >250 | >250 |

(continued)

| Compound | Structure | Without colistin $IC_{50}$ (μM) | Synergy with colistin (8 μg/ml) | |
|---|---|---|---|---|
| | | | $IC_{50}$ (μM) | $IC_{90}$ (μM) |
| BBN152 | 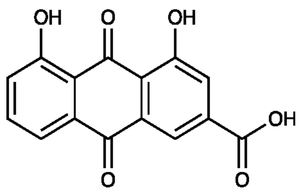 | >250 | >250 | >250 |
| BBN153 | 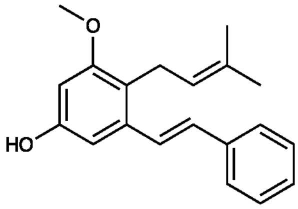 | >250 | 15.625 | >250 |
| BBN154 | 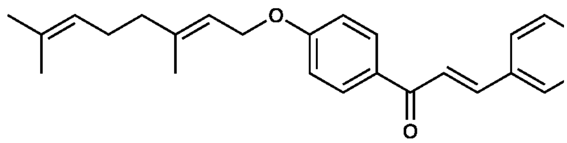 | >250 | >250 | >250 |
| [1] The values of $IC_{50}$ and $IC_{90}$ indicate the minimum concentration of compound able to cause at least 50% and 90% respectively of inhibition of bacterial growth. | | | | |

*Evaluation of the antibacterial activity of the compounds identified by in silico screening*

The compounds identified by *in silico* screening were tested for their antibacterial activity (in the absence of colistin) and for their synergistic or adjuvant activity with colistin against a colistin-resistant strain of *P. aeruginosa* grown in the absence or presence of a sub-inhibitory concentration of colistin (8 μg/ml). As reported in Table 1, none of the compounds showed antibacterial activity per se against *P. aeruginosa.* On the contrary, some compounds were found to inhibit bacterial growth in the presence of colistin (BBN79, BBN118, BBN120, BBN147, BBN149 and BBN153) with $IC_{50}$ values (concentration of compound necessary to inhibit bacterial growth of at least 50%) between 16 and 250 μM (Table 1), thus suggesting their synergistic or adjuvant activity with this compound. Among the various compounds with synergistic or adjuvant activity, only the BBN149 compound caused a complete inhibition of the growth of the reference strain, with an $IC_{90}$ value (concentration of compound necessary to inhibit bacterial growth of at least 90%) equal to about 30 μM (Table 1).

*Isolation of BBN149 analogues with diterpene structure extracted from Fabiana densa var. ramulosa and activity essays*

The BBN149 diterpene was obtained by solid-liquid extraction from the aerial parts of *Fabiana densa var. ramulosa,* which were collected and identified by the Department of Pharmacological and Toxicological Chemistry, University of Chile.

The aerial parts (600 g) were left to macerate in acetone for 24h. Subsequently, the insoluble fraction was separated by filtration and new solvent was added to the solid matrix in order to increase the extraction efficiency. This operation was carried out several consecutive times. The different soluble fractions were collected together and evaporated under vacuum. The filtrate (100g) was purified by flash chromatography. As a mobile phase, a mixture of hexane:ethyl acetate (EtOAc) was used, starting from 100% of hexane up to a ratio of 97:3 of the eluent system which allowed the isolation of the alcoholic derivative FDA (10%) and a fraction (15g) composed of a mixture of three products. This latter fraction was subjected to a second chromatographic purification by using a gradient elution. A chloroform ($CHCl_3$):methanol ($CH_3OH$) mixture was used as the mobile phase. Using a ratio 98:2 of the eluent system the succinic derivative FDS was isolated with a yield of 20%. The increase in polarity of the mobile phase, obtained using a ratio between the two solvents equal to 97:3, allowed the elution of the malonic derivative FDM with a yield equal to 10%.

The further increase in polarity through the use of an eluent mixture $CHCl_3$:$CH_3OH$:formic acid in a ratio 97:3:1% allowed the isolation of the oxalic derivative BBN149 with a yield equal to 5%.

The compounds thus obtained (BBN149, FDA, FDM and FDS) were tested for their antibacterial and synergistic or adjuvant activity with colistin as previously described. According to the results previously obtained for the compound BBN149 (Table 1), none of the compounds showed antibacterial activity per se, while three out of four compounds (BBN149, FDM and FDS) were able to inhibit bacterial growth in the presence of colistin (Table 2). Compared to the reference compound BBN149, however, the FDM and FDS compounds showed a higher $IC_{50}$ and an inability to cause complete inhibition of bacterial growth (values of $IC_{90}$ > 250 μM). Table 2. Structure and synergistic activity with colistin of derivatives of natural origin of the diterpene BBN149 (FDA, FDS and FDM). Data obtained with the *P. aeruginosa* PA14 $Col^R5$ strain, for which the MIC and $IC_{90}$ of colistin is equal to 64 μg/ml.

| Compound | Structure | Without colistin $IC_{50}$ (μM) | Synergy with colistin (8 μg/ml) | |
|---|---|---|---|---|
| | | | $IC_{50}$ (μM) | $IC_{90}$ (μM) |
| FDA | 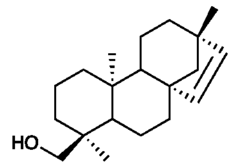 | >250 | >250 | >250 |
| BBN149 | 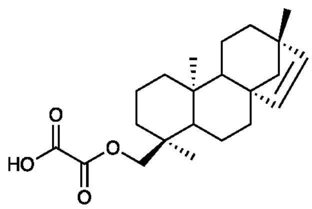 | >250 | 15.625 | 31.25 |
| FDS | 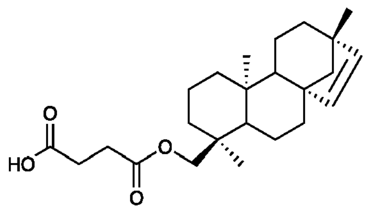 | >250 | 31.25 | >250 |
| FDM | 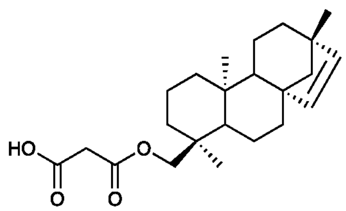 | >250 | 62.5 | >250 |

The structural identity of the isolated compounds was determined by nuclear magnetic resonance spectroscopy ($^1H$-NMR and $^{13}C$-NMR) and mass spectrometry (MS).

*Characterization of the components of the extract*

**Ent-beyer-15-en-18-ol (FDA)**

White solid (yield 10%); m.p. 110°C ± 0.5 °C $[\alpha]_D$ +29.7° ($CHCl_3$). $^1H$ NMR ($CDCl_3$, 400 MHz): δ (ppm) = 5.69 (d, 1H, J = 5.7 Hz, H-15); 5.45 (d, 1H, J = 5.6 Hz, H-16); 3.41 (d, 1H, J = 10.8 Hz, H-18a); 3.11 (d, 1H,J = 10.8 Hz, H-18b); 0.99 (s, 3H, $CH_3$-17); 0.78 (s, 3H, $CH_3$-19); 0.77 (s, 3H, $CH_3$-20); $^{13}C$ NMR ($CDCl_3$, 100 MHz): δ (ppm) = 136.59, 135.38, 72.48, 61.34, 52.94, 49.19, 49.14, 43.81, 38.90, 37.74, 37.28, 37.16, 35.54, 33.34, 25.11, 20.38, 20.02, 18.12, 17.96, 15.76.

ESI-MS (positive) m/z: $[M+Na]^+$ calculated for $C_{20}H_{32}ONa$ 311.25, found 311.3.

**Ent-beyer-15-en-18-O-succinate (FDS)**

Brown solid (yield 20%); m.p. 107.5 °C ± 0.5 °C. [α ]$_D$ +14.6° ($CHCl_3$). $^1H$ NMR ($CDCl_3$, 400 MHz): δ (ppm) = 5.67 (d, 1H, J = 5.6 Hz, H-15); 5.45 (d, 1H, J = 5.6 Hz, H-16); 3.88 (d, 1H, J = 10.8 Hz, H-18a); 3.68 (d, 1H, J = 10.08 Hz, H-18b); 2.67 (m, 4H, HOOC-$CH_2CH_2$-COOR); 0.99 (s, 3H, $CH_3$-17); 0.83 (s, 3H, $CH_3$-19); 0.77 (s, 3H, $CH_3$-20). $^{13}C$ NMR ($CDCl_3$, 100 MHz): δ (ppm) = 177.44, 172.28, 136.60, 135.35, 73.61, 61.24, 52.93, 50.01, 49.05, 43.75, 38.74, 37.29, 37.05, 36.70, 36.01, 33.27, 29.20, 29.06, 25.06, 20.30, 20.20, 17.91, 17.80, 15.63.

ESI-MS (negative) m/z: [M-H]- calculated for $C_{24}H_{35}O_4$ 388.54, found 387.5; $[M+Cl]^-$ claculated for $C_{24}H_{36}O_4Cl$ 423.40, found 423.20.

**Ent-beyer-15-en-18-O-malonate (FDM)**

Green oil (yield 10%); oily. [α ]$_D$ +25.8° ($CHCl_3$). $^1H$ NMR ($CDCl_3$, 400 MHz): δ (ppm) = 5.67 (d, 1H, J = 5.6 Hz H-15); 5.45 (d, 1H, J = 5.6 Hz, H-16); 3.98 (d, 1H, J = 10.8 Hz, H-18b); 3.75 (d, 1H, J = 10.08 Hz, H-18a); 3.44 (s, 2H, HOOC-$CH_2$-COOR); 0.99 (s, 3H, $CH_3$-17); 0.85 (s, 3H, $CH_3$-19); 0.77 (s, 3H, $CH_3$-20). $^{13}C$ NMR ($CDCl_3$, 100 MHz): δ (ppm) = 169.29, 167.81, 136.66, 135.19, 74.66, 61.23, 52.92, 49.88, 49.03, 43.76, 40.53, 38.69, 37.31, 36.97, 36.77, 35.95, 33.26, 25.04, 20.30, 20.24, 17.85, 17.72, 15.62.

ESI-MS (negative) m/z: $[M-H]^-$ calculated for $C_{23}H_{33}O_4$ 373.51; found 373.1.

**Ent-beyer-15-en-18-O-ossalate (BBN149)**

White solid (yield 5%) m.p. 169.5 °C ± 0.5 °C. [α ]$_D$ +10° ($CHCl_3$). $^1H$ NMR ($CDCl_3$, 400 MHz): δ (ppm) = 5.67 (d, 1H, J = 5.7 Hz, H-15,); 5.46 (d, 1H, J = 5.7 Hz, H-16,); 4.10 (d, 1H, J = 10.8 Hz, H-18a); 3.90 (d, 1H, J = 10.8 Hz, H-18b); 0.99 (s, 3H, $CH_3$-17); 0.91 (s, 3H, $CH_3$-19); 0.79 (s, 3H, $CH_3$-20). $^{13}C$ NMR ($CDCl_3$, 100 MHz): δ (ppm) = 158.65, 157.77, 136.74, 135.13, 76.54, 61.17, 52.83, 50.08, 49.01, 43.77, 38.57, 37.36, 36.98, 36.94, 35.92, 33.21, 25.03, 20.35, 20.29, 17.78, 17.62, 15.63.

ESI-MS (negative) m/z: $[M-H]^-$ calculated for $C_{22}H_{31}O_4$ 360.49; found 359.4.

*Characterization of the compounds of natural origin*

The chemical identity of the compounds examined was verified by Nuclear Magnetic Resonance (NMR). The results obtained were found to be in agreement with those reported in the literature.

Compound BBN36 (aloin or (10S)-1,8-dihydroxy-3-(hydroxymethyl)-10-[(2S, 3R, 4R, 5S, 6R)-3,4,5-trihydroxy-6-(hydroxymethyl)oxan-2-yl]-10 H-anthracen-9-one) the NMR analysis is in accordance with what reported in the literature (Jang Hoon K. et al, Journal of Enzyme Inhibition and Medicinal Chemistry, 2017, vol. 32, 78-83).

BBN53 compound (chlorogenic acid or (1S, 3R, 4R, 5R)-3-[(E)-3-(3,4-dihydroxyphenyl)prop-2-enoyl]oxy-1,4,5-trihydroxycyclohexane-1-carboxylic acid) NMR analysis is in agreement with what reported in the literature (W.Khoo et al., 2015).

BBN79 compound (verbascoside or [(2R, 3R, 4R, 5R, 6R)-6-[2-(3,4-dihydroxyphenyl)ethoxy]-5-hydroxy-2-(hydroxymethyl)-4-[(2S, 3R , 4R, 5R, 6S)-3,4,5-trihydroxy-6-methyloxan-2-yl] oxyoxane-3-yl] (E)-3-(3,4-dihydroxyphenyl)prop-2-enoate) the NMR analysis is in agreement with what reported in the literature (Venditti A. et al, 2013).

Compound BBN101 (floretin or 3-(4-hydroxyphenyl)-1-(2,4,6-trihydroxyphenyl)propan-1-one) the NMR analysis is in agreement with what reported in the literature (Qingwen Hu et al., 2018).

BBN118 compound (piscidone or 3-[3,4-dihydroxy-6-methoxy-2-(3-methylbut-2-enyl)phenyl]-5,7-dihydroxycromen-4-one) the NMR analysis is in agreement with what reported in literature (Tahara S. et al., 1991).

Compound BBN119 (Rheediaxantone A or 11,22-dihydroxy-7,7,19,19-tetramethyl-2,8,20-trioxapentacyclo [12.8.0.03,120,04,90,016,21]docosa-1(14), 3(12), 4(9), 5, 10, 15, 17, 21-octaen-13-one) the NMR analysis is in agreement with what reported in the literature (Delle Monache F. et al., 1981).

Compound BBN120 (rheediaxanthone B or NMR analysis is in accordance with what reported in the literature (Delle Monache F. et aL, 1981).

Compound BBN135 (harmane or 1-methyl-9H-pyrido[3,4-b]indole) the NMR analysis is in agreement with what reported in the literature (Z. Zhao et al., 2019).

BBN139 compound (loganine or methyl (1S, 4aS, 6S, 7R, 7aS)-6-hydroxy-7-methyl-1-[(2 S, 3 R, 4 S, 5S, 6R)-3,4,5-trihydroxy-6-(hydroxymethyl)oxan-2-yl]oxy-1, 4a, 5,6,7,7a-haxidrocrociclopenta [c] piran-4-carboxylate) the NMR analysis is in agreement with what reported in the literature (Garaev et al., 2014).

Compound BBN145 (3-hydroxy-4-methoxy cinnamic acid) the NMR analysis is in agreement with what reported in the literature (Set Byeol K. et al., 2017).

Compound BBN146 (6-methoxy-7-O-geranyl-coumarin) the NMR analysis is in agreement with what reported

in the literature (Fiorito S. et al., 2018).

Compound BBN147 (6-prenyl-aromadendrine) the NMR analysis is in agreement with what reported in the literature (Toshio F. et al., 1993).

Compound BBN148 (vismiafenone b or [5,7-dihydroxy-2,2-dimethyl-8-(3-methylbut-2-enyl) cromen-6-yl]-phenylmethanone) the NMR analysis is in agreement with what reported in the literature (Delle Monache et al., 1980).

Compound BBN149 (ent-beyer-15-en-18-O-oxalic acid) the NMR analysis is in agreement with what reported in the literature (Erazo S. et al., 2002).

Compound BBN151 (physcione or 1,8-dihydroxy-3-methoxy-6-methyl-anthracene-9,10-dione) the NMR analysis is in agreement with what reported in the literature (Delle Monache F. et al., 1979)

Compound BBN152 (rhein or 4,5-dihydroxy-9,10-dioxoanthracene-2-carboxylic acid) the NMR analysis is in agreement with what reported in the literature (Manshuo L. et al., 2017).

Compound BBN153 (longistiline c or 3-methoxy-4-(3-methylbut-2-enyl)-5-[(E)-2-phenylethylene]phenol) the NMR analysis is in agreement with what reported in the literature (Xing-Yue J. Et al., 2016).

Compound BBN154 (calcone19 or 4'-O-geranil-calcone) the NMR analysis is in agreement with what reported in the literature (Guglielmi P. et al., 2019).

*Design and synthesis of BB149 derivatives and activity assays*

The results obtained from the microbiological tests suggested the promising role of the diterpene ent-beyerenic scaffold in modulating colistin resistance in colistin-resistant bacterial infections. Given that the BBN149 molecule emerged from the first screening as a promising hit, a first generation of diterpene derivatives was designed and synthesized with the aim of increasing the co-adjuvant ability the action of colistin and delineating the SAR (structure-activity relationship).

The ent-beyerenic scaffold differs from the ent-kaurenic one in the presence of an exocyclic double bond:

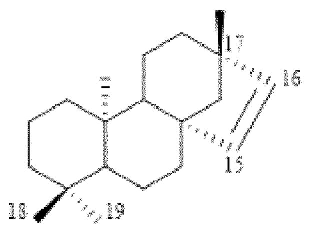

ent-beyer-15-ene

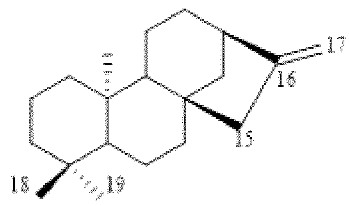

ent-kaur-16-ene

Given the possibility of obtaining diterpenes with an ent-beyeranic structure, starting from diterpenes with an ent-kaurenic structure characteristic of the diterpenes of the *Stevia rebaudiana var. ramulosa,* analogues of the compound BBN149 have been designed wherein the double bond is not present at positions 15 and 16 and the chiral carbon in position 4 has a different stereochemistry (R instead of S).

Diterpene derivatives have been tested for their antibacterial and synergistic activity with colistin as previously described. No compound showed antibacterial activity per se, while three compounds (SR4, SR8 and SR10) were able to inhibit bacterial growth in the presence of colistin, with $IC_{50}$ values around 15-30 $\mu$M (Table 3). No compound was able to completely inhibit bacterial growth ($IC_{90}$ values > 250 $\mu$M).

Table 3. Structure and synergistic or adjuvant activity with colistin of synthetic analogues of diterpene BBN149 (SR1-SR10). Data obtained with the *P. aeruginosa* PA14 Col[R]5 strain, for which the MIC and $IC_{90}$ of colistin is equal to μg/ml.

| Compound | Structure | Without colistin $IC_{50}$ (μM) | Synergy with colistin (8 μg/ml) | |
|---|---|---|---|---|
| | | | $IC_{50}$ (μM) | $IC_{90}$ (μM) |
| SR1 | 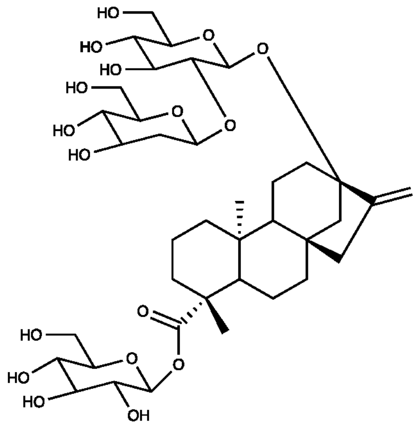 | >250 | >250 | >250 |
| SR2 | 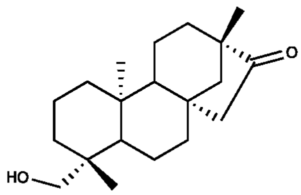 | >250 | >250 | >250 |
| SR3 | 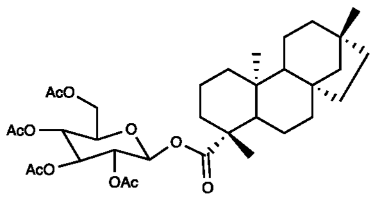 | >250 | >250 | >250 |
| SR4 | 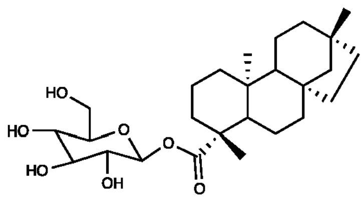 | >250 | 15.625 | >250 |
| SR5 | 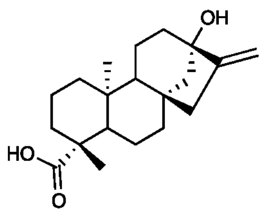 | >250 | >250 | >250 |

(continued)

| Compound | Structure | Without colistin $IC_{50}$ (μM) | Synergy with colistin (8 μg/ml) | |
|---|---|---|---|---|
| | | | $IC_{50}$ (μM) | $IC_{90}$ (μM) |
| SR6 | 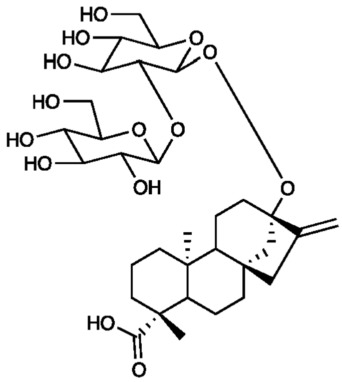 | >250 | >250 | >250 |
| SR7 | 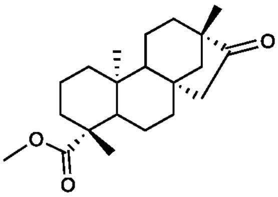 | >250 | >250 | >250 |
| SR8 | 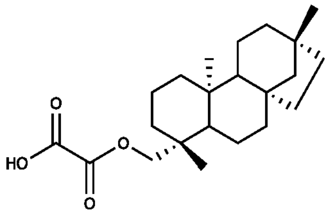 | >250 | 31.25 | >250 |
| SR9 | 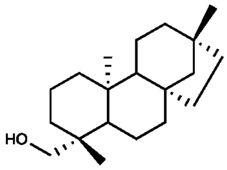 | >250 | >250 | >250 |
| SR10 | 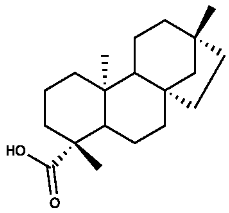 | >250 | 31.25 | >250 |
| FDO-H | 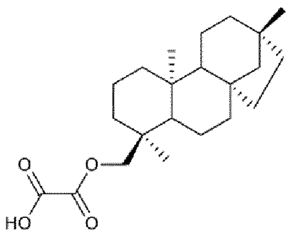 | <250 | 7.812 | 15,625 |

*Chemical products, reagents and methods of analysis*

All reagents and solvents are commercially available and have been used without further purification.

Silica gel (230-400 mesh) was used for purification by flash column chromatography. All reactions were monitored by thin layer chromatography (TLC) and F254 fluorescence silica gel plates (Sigma-Aldrich 99569) were used. The melting points were determined with a Buchi Melting Point B - 454 apparatus. The $^{1}H$ and $^{13}C$ NMR spectra were recorded with a Bruker 400 Ultra Shield™ instrument (400 MHz for $^{1}H$ NMR and 100 MHz for $^{13}C$ NMR), using tetramethyl silane (TMS) as standard. Chemical shifts are reported in parts per million (ppm). The multiplicities were reported as follows: singlet (s), doublet (d), triplet (t) and multiplet (m). Mass spectrometry was performed with the Thermo Finnigan LXQ linear ion trap mass spectrometer, equipped with electrospray ionization (ESI). High resolution mass spectra (HR-MS) were recorded with a Bruker BioApex Fourier transform ion cyclotron resonance (FT-ICR).

Synthetic procedures

Synthesis of the compound SR2 (reference example) The compound SR1 (SIG MA-ALDRICH 260-975-5) (8.68 mmol, 7.0 g) is treated with hydrobromic acid (48% HBr in water) (21 ml) and the solution, which takes on a brown colour, is left under stirring at room temperature for 16 hours. Subsequently, the precipitate is filtered and solubilized in AcOEt. The organic phase is extracted once with water and once with brine, dehydrated with anhydrous $Na_2SO_4$ and concentrated at a reduced pressure. The compound SR2 (7.72 mmol, 2.45 g) is obtained by crystallization with $CH_3OH$. (Lohoelter C. et al., 2013; Avent et al., 1989).

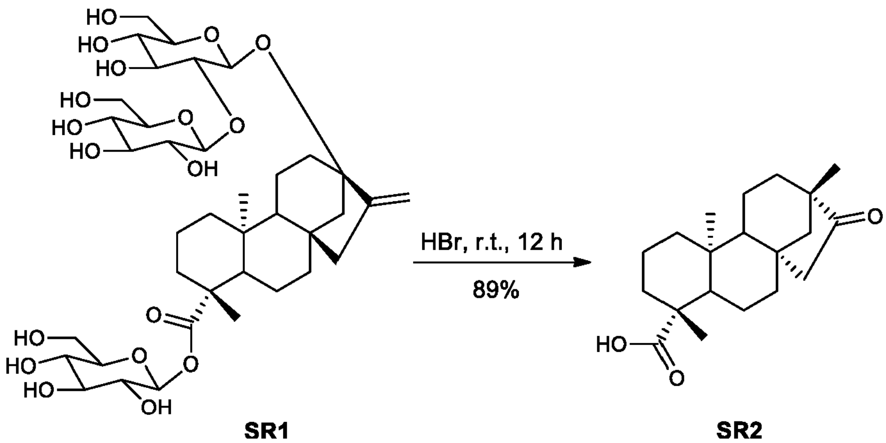


SR1 SR2

**Scheme 1**

**SR2**

Brown powder (yield 89%); m.p. 230 °C ± 0.5 °C. [α ]$_D$ -69.3° (EtOH). $^{1}H$ NMR ($CDCl_3$, 400 MHz): δ (ppm) = 2.64 (dd, 1H, J = 18.6 Hz e J = 3.7 Hz, 1H, H-15a); 2.07 (d, 1H, J = 13.3 Hz, H-3eq); 1.93-1.32 (m, 13H); 1.25 (s, 3H, $CH_3$-18); 1.23-1.01 (m, 4H); 0.97 (s, 3H, $CH_3$-17); 0.96-079 (m, 1H, H-1ax); 0.78 (s, 3H, $CH_3$-20); $^{13}C$ NMR ($CDCl_3$, 100 MHz): δ (ppm) = 222.90, 183.50, 57.17, 54.90, 54.44, 48.89, 48.61, 43.81, 41.60, 39.90, 39.63, 38.34, 37.84, 37.46, 29.11, 21.77, 20.49, 19.99, 19.01, 13.48.

ESI-HRMS (positive) m/z: $[M+Na]^+$ calculated for $C_{20}H_{30}O_3Na$ 341.20872; found 341.20902.

Synthesis of compound SR10

A solution containing SR2 (3.14 mmol, 1.0 g), triethylene glycol (12.5 ml), hydrazine (2.5 ml) and potassium hydroxide (KOH) (22.3 mmol, 1.25 g) is distilled at 180 °C, until the removal of a volume of about 1.25 ml. After the distillation is over, the Dean-Stark is removed and the reaction is left under stirring to reflux for 22 hours at 180 °C and, subsequently, for 2 hours at 200 °C. Subsequently, the reaction is brought to room temperature and 162.5 ml of distilled water are added. The solution is neutralized with glacial acetic acid ($CH_3COOH$) 1N. The precipitate is filtered and solubilized in diethyl ether ($Et_2O$). Finally, this solution is extracted 2 times with water, dehydrated with anhydrous $Na_2SO_4$ and concentrated under reduced pressure, thus obtaining SR10 (0.911 mmol, 277 mg). (Mosetting E. et al., 1955; Yang et al., 2012).

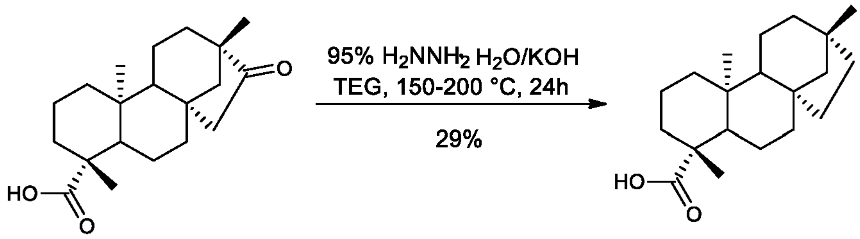


SR2 SR10

Scheme 2

**SR10**

White powder (yield 29%); melting point: 190 °C ± 0.5 °C. $[\alpha]_D$ -42.0° ($CHCl_3$). $^1H$ NMR ($CDCl_3$, 400 MHz): δ (ppm) = 2.13 (d, 1H, J = 12 Hz, H-3eq); 2.02-1.24 (m, 15H); 1.22 (s, 3H, $CH_3$-18); 1.18-0.95 (m, 5H); 0.93 (s, 3H, $CH_3$-17); 0.89 (m, 1H, H-1ax); 0.84 (s, 3H, $CH_3$-20). $^{13}C$ NMR ($CDCl_3$, 100 MHz): δ (ppm) = 184.04, 60.54, 57.52, 56.33, 45.13, 43.83, 41.54, 40.19, 40.18, 39.45, 38.36, 37.96, 37.69, 33.63, 29.19, 27.26, 21.92, 20.95, 19.09, 14.32.

ESI-HRMS (negative) m/z: $[M-H]^-$ calculated for $C_{20}H_{31}O_2$ 303.23295; found 303.23288.

Synthesis of compound SR3 (reference example)

To a solution containing SR10 (0.986 mmol, 300 mg) in dichloromethane ($CH_2Cl_2$) (6.73 ml) and water (1.79 ml), tetrabutylammonium bromide (TBAB) (0.02 mmol, 6.72 mg), potassium carbonate ($K_2CO_3$) (3.26 mmol, I450 mg) and acetobromo-α-D-glucose (1.36 mmol, 560 mg) are added. The solution is left under stirring to reflux for 24 hours at a temperature of 50 °C. Subsequently, the aqueous phase is extracted with $CH_2Cl_2$ and the organic phases, in turn, are extracted twice with water, once with brine and finally dehydrated with anhydrous $Na_2SO_4$. Following evaporation of the solvent under reduced pressure, the compound SR3 (0.572 mmol, 363 mg) was obtained. (Klucik J. et al., 2011; Chaturvedula et al., 2011; Yang et al., 2012).

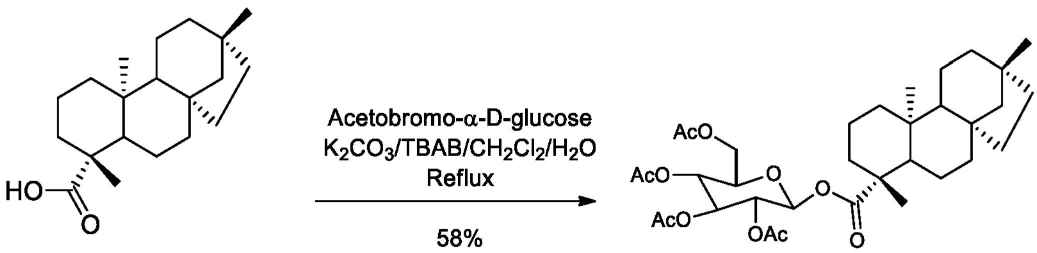


SR10 SR3

Scheme 3

**SR3**

Brown powder (yield 58%); m.p. 140 °C ± 0.5 °C. $[\alpha]_D$ -8.7°($^1H$ NMR ($CD_3OD$, 400 MHz): δ (ppm) =5.82 (d, J = 8.4 Hz, 1H, H-1'); 5.34 (t, 1H, J = 9.4 Hz, H-3'); 5.12-5.05 (m, 2H, H-2'e H-4'); 4.33 (dd, 1H, J = 12.1 Hz, J = 4.8 Hz, H-6'); 4.02 (dd, 1H, J = 12.0 Hz, J = 2.4 Hz, H-6"); 4.02-3.98 (m, 1H, H-5'); 2.04 (s, 3H, $CH_3CO$); 2.02 (s, 6H, 2 × $CH_3CO$); 1.98 (s, 3H, $CH_3CO$); 1.80-1.25(m, 16H); 1.17 (s, 3H, $CH_3$-17); 1.41-0.97 (m, 5H); 0.94 (s, 3H, $CH_3$-18); 0.77 (s, 3H, $CH_3$-20). $^{13}C$ NMR ($CD_3OD$, 100 MHz): δ (ppm) = 177.16, 172.23, 171.55, 171.23, 170.79, 92.50, 74.54,73.57, 71.77, 69.45, 62.66, 58.53, 58.48,57.48, 46.16, 45.18, 42.51, 41.12, 41.09, 40.34, 39.29, 39.03, 38.52, 34.82, 29.23, 27.51, 22.96, 21.89, 20.93, 20.52, 14.42.

ESI-HRMS (positive) m/z: $[M+Na]^+$ calculated for $C_{34}H_{50}O_{11}Na$ 657.32453; found 657.32481.

Synthesis of compound SR4

A solution of compound SR3 (0.495 mmol, 314 mg) in $CH_3OH$: $H_2O$: hexane (10: 2: 1) at 10% of $Et_3N$ (7.6 ml) is stirred at room temperature for 48 hours, at the end of which the solution is concentrated under pressure and the

residue obtained, the compound SR4 (0.511 mmol, 238 mg), is crystallized with $Et_2O$ at room temperature. (Ouilmi D. et al., 1995; Chaturvedula et al., 2011; Yang et al., 2012).

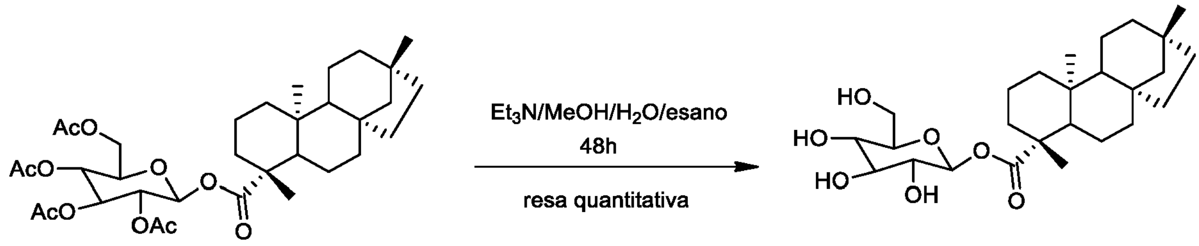


SR3

SR4

## Scheme 4

**SR4**

White powder (quantitative yield); m.p. 160 °C ± 0.5 °C. [α ]$_D$ -22.7° (MeOH). $^1$H NMR ($CD_3OD$, 400 MHz): δ (ppm) = 5.42 (d, 1H, J = 8.3 Hz, H-1'); 3.83 (dd, 1H, J = 12.1 Hz, J = 1.6 Hz, H-6'); 3.67 (dd, 1H, J = 11.6 Hz, J = 4.4 Hz, H-6"); 3.40-3.34 (m, 4H, H-2', H-3', H-4', H-5'); 2.20-2.17 (d, 1H, J = 13.2, H-3eq); 2.09-2.03 (m, 1H, H-5eq); 1.91-1.34 (m, 14H); 1.21 (s, 3H, $CH_3$-17); 1.18-0.97 (m, 5H); 0.94 (s, 3H, $CH_3$-18); 0.87 (s, 3H, $CH_3$-20). $^{13}$C NMR ($CD_3OD$, 100 MHz): δ (ppm) = 178.27, 95.55, 78.69, 78.66, 74.03, 71.11, 62.42, 59.10, 58.56, 57.57, 46.25, 45.10, 42.75, 41.29, 41.23, 40.32, 39.37, 39.04, 38.53,34.51, 29.24, 27.60 22.86, 21.93, 20.07, 14.42.

ESI-HRMS (positive) m/z: [M+Na]$^+$ calculated for $C_{26}H_{42}O_7Na$ 489.28227; found 489.28264.

Synthesis of compound SR9 (reference example)

To a solution of SR10 (1 mmol, 304 mg) in tetrahydrofuran (THF) (0.0854 n/l, 11.70 ml), lithium tetrahydroaluminate $LiAlH_4$ (9 mmol, 4.5 ml) is added dropwise. The reaction is left to reflux for about three hours at the end of which the excess of $LiAlH_4$ is eliminated by adding EtOAc and 20 drops of a saturated solution of Rochelle Salt. The solution is evaporated under pressure to eliminate the excess of THF, extracted with EtOAc and dehydrated with anhydrous $Na_2SO_4$. The SR9 compound (0.82 mmol, 238 mg) was obtained with a yield of 82%. (Batista et al., 2007; Murillo et al., 2019; Yang et al., 2012).

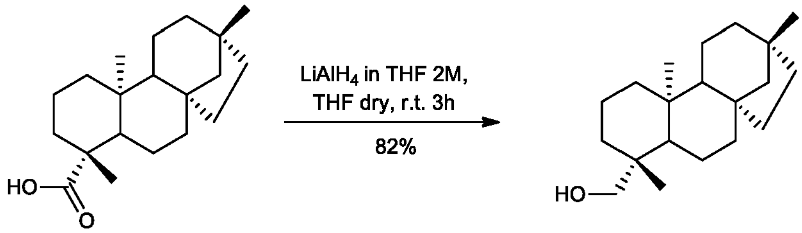


SR10

SR9

## Scheme 5

**SR9**

White powder (yield 82%); m.p. 115 °C ± 0.5 °C; [α ]$_D$ -0.7° ($CHCl_3$). $^1$H NMR ($CDCl_3$, 400 MHz): δ (ppm) = 3.75 (d, 1H, J = 8 Hz, H-19b); 3.41 (d, 1H, J = 8 Hz, H-19a,); 1.75 (d, 1H, J = 12Hz, H-3eq); 1.72-1.16 (m, 19H); 0.95 (s, 3H, $CH_3$-17); 0.93 (s, 3H, $CH_3$-19); 0.89 (s, 3H, H-20). $^{13}$C NMR ($CDCl_3$, 100 MHz): δ (ppm) = 65.82, 57.78, 57.32, 57.14, 45.09, 41.78, 40.10, 39.91, 39.42, 38.67, 37.75, 37.69, 35.69, 33.74, 27.30, 27.21, 20.74, 20.50, 18.25, 15.85.

ESI-MS (positive) m/z: [M+Na]$^+$ calculated for $C_{20}H_{34}ONa$ 313.26; found 313.7.

Synthesis of compound SR8

To a solution containing the compound SR9 (0.207 mmol, 60 mg) in $Et_2O$ (0.192 mmol/ml, 1.08 ml) at 0 °C,

oxalyl chloride (0.414 mmol, 0.207 ml) is added dropwise (ratio between starting substrate and reactive 1:2). The solution is left under stirring for 30 minutes at room temperature. The reaction is then switched off by adding $H_2O$ until there is no more effervescence. The aqueous solution is extracted with $Et_2O$ and the organic phase thus obtained is washed twice with water and once with brine, and dehydrated with $Na_2SO_4$. The residue is evaporated under pressure and purified through a flash chromatographic column using an eluent mixture $CHCl_3$: $CH_3OH$:HCOOH in a ratio 98 : 2 : 1%. The SR8 compound (0.190 mmol; 69 mg) was obtained with a yield of 92%. (Zhang X. et al., 2016).

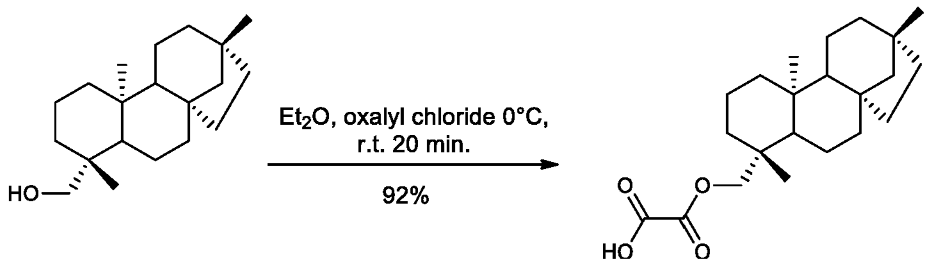


SR9 SR8

**Scheme 6**

**SR8**

Oil (yield 92%); oily. [α ]$_D$ -14.3° ($CHCl_3$). $^1$H NMR ($CDCl_3$, 400 MHz): δ (ppm) = 4.52 (d, 1H, J = 10.8 Hz, H-19a); 4.10 (d, 1H, J = 10.8 Hz, H-19b); 2.04-1.3 (m, 17H); 1.16-1.03 (m, 5H); 1.00 (s, 3H, $CH_3$-17); 0.94 (s, 3H, $CH_3$-19); 0.92 (s, 3H, H-20). $^{13}$C NMR ($CDCl_3$, 100 MHz): δ (ppm) = 158.59, 158.18, 71.10, 57.70, 57.15, 57.01, 45.03, 41.51, 40.03, 39.52, 39.42, 37.67, 37.66, 37.37, 36.06, 33.69, 27.42, 27.26, 20.72, 20.35, 18.05, 15.86.

ESI-MS (positive) m/z: $[M+Na]^+$ calculated for $C_{22}H_{34}O_4Na$ 385.25; found 385.3.

Synthesis of SR6 (reference example)

The SR1 compound (0.62 mmol, 500 mg) is treated with a 10% solution of potassium hydroxide (KOH) (12.5 ml). The reaction is left under stirring for one hour at a temperature of 100 °C. Subsequently, the reaction is cooled to room temperature, neutralized with a solution of $CH_3COOH$ 1N and concentrated under reduced pressure. The SR6 compound (0.589 mmol, 390 mg) was obtained with a yield of 95% by crystallization with $CH_3OH$. (Wood JR et al., 1955, Chaturvedula et al., 2011).

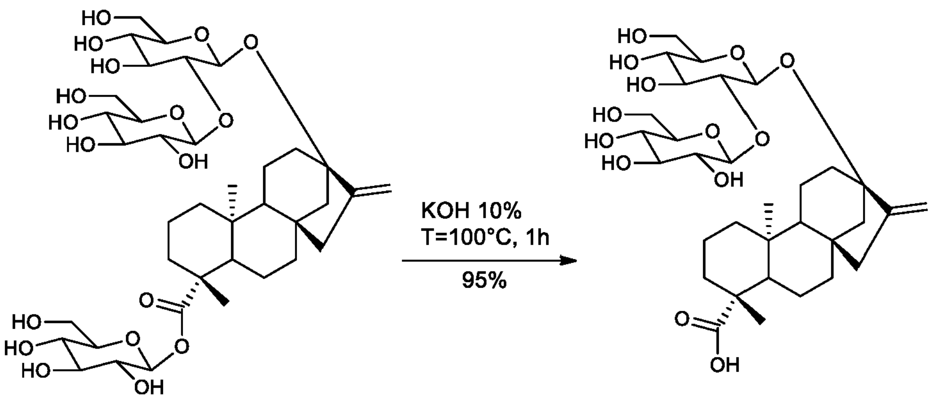


SR1 SR6

**Scheme 7**

**SR6**

White powder (yield 95 %); m.p. 213 °C ± 0.5 °C. [α ]$_D$ -32.5° (MeOH). $^1$H NMR (DMSO-$d_6$, 400 MHz): δ (ppm) = 5.08 (s, 1H, H-17); 4.74 (s, 1H, H-17b); 4.46 (d, 1H, J = 8 Hz, H-1'); 4.35 (d, 1H, J = 8 Hz, H-1"); 3.63-2.98 (m, 12 H); 2.17-1.22 (m, 17H); 1.07 (s, 3H, H-18); 0.89 (s, 3H, H-20);0.80-0.70 (m, 2H,); $^{13}$C NMR (DMSO-$d_6$, 100 MHz): δ (ppm) = 179.50, 153.41, 104.75, 103.97, 96.36, 84.90, 82.45, 76.98, 76.63, 76.12, 76.02, 75.51, 69.87, 69.75, 60.92, 60.62,

56.56, 53.39, 47.22, 43.87, 43.09, 41.93, 41.27, 41.22, 40.66, 38.37, 36.13, 29.21, 21.95, 19.96, 19.21, 15.35.

ESI-HRMS (positive) m/z: $[M+Na]^+$ calculated for $C_{32}H_{50}O_{13}Na$ 665.31436; found 665.31488.

Synthesis of compound SR7 (reference example)

The SR2 compound (0.943 mmol, 300 mg) is treated, at 0 ° C, with thionyl chloride ($SOCl_2$) (13.6 ml) and anhydrous dimethylformamide (DMF) (0.3 ml). The solution is left under stirring for 2 hours at room temperature. Subsequently, the solvent is evaporated under reduced pressure, and anhydrous $CH_3OH$ (54.5 ml) and triethylamine ($Et_3N$) (12.3 ml) are added at 0 ° C. The solution is left under stirring for 2 hours at room temperature and, subsequently, the residue obtained by evaporating the solvent is solubilized in $CH_2Cl_2$. The organic phase is extracted three times with brine and dehydrated with anhydrous $Na_2SO_4$, leaving it stirring overnight. The SR7 compound (0.943 moles, 313 mg) was obtained with a quantitative yield (Batista et al., 2007; Avent et al., 1989)

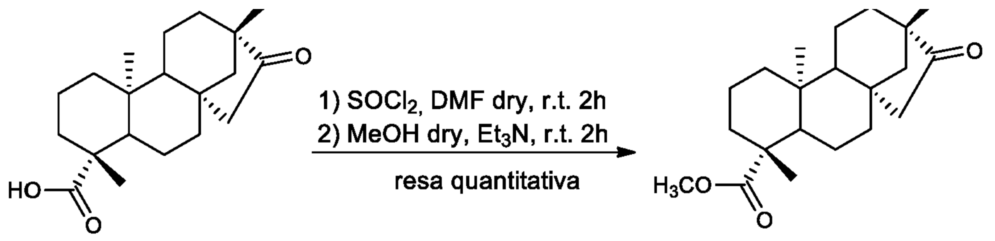


SR2 SR7

**Scheme 8**

**SR7**

Yellow powder (quantitative yield); m.p. 184 °C ± 0.5 °C. $[\alpha]_D$ -46.0° ($CHCl_3$). $^1H$ NMR ($CDCl_3$, 400 MHz): $\delta$ (ppm) = 3.63 (s, 3H, $COOCH_3$); 2.63 (dd, 1H, J = 18.6 Hz, J = 3.8 Hz, H-15ax); 2.18 (d, 1H, J = 14.1 Hz, H-3eq); 2.02-1.33 (m, 15H); 1.19 (s, 3H, $CH_3$-18); 1.14-0.10 (m, 5H); 0.97 (s, 3H, $CH_3$-17); 0.89 (m, 1H, H-1ax); 0.68 (s, 3H, $CH_3$-20). $^{13}C$ NMR ($CDCl_3$, 100 MHz): $\delta$ (ppm) = 177.98, 57.22, 54.89, 54.47, 51.40, 48.86, 48.62, 43.94, 41.64, 39.97, 39.60, 38.10, 37.47, 29.00, 21.86, 20.48, 20.00, 19.10, 13.32.

ESI-HRMS (positive) m/z: $[M+Na]^+$ calculated for $C_{21}H_{32}O_3Na$ 355.22437; found 355.22466.

Synthesis of compound SR5 (reference example)

A solution containing SR1 (1.36 mmol, 1.1 g) and sodium periodate ($NaIO_4$) (7 mmol, 1.5 g) in water (75 ml) is left under stirring at room temperature for 16 hours. Subsequently KOH (134 mmol, 7.5 g) is added to the solution, which is left under stirring to reflux for 1 hour. Thereafter, the solution is neutralized at room temperature with $CH_3COOH$. The aqueous phase is extracted with $Et_2O$, while the organic phase is extracted with water and dehydrated with anhydrous $Na_2SO_4$. The compound SR5 (1.02 mmol, 324.58 mg) is obtained with a yield of 75% by crystallization with $CH_3OH$. (Batista et al., 2007; Avent et al., 1989).

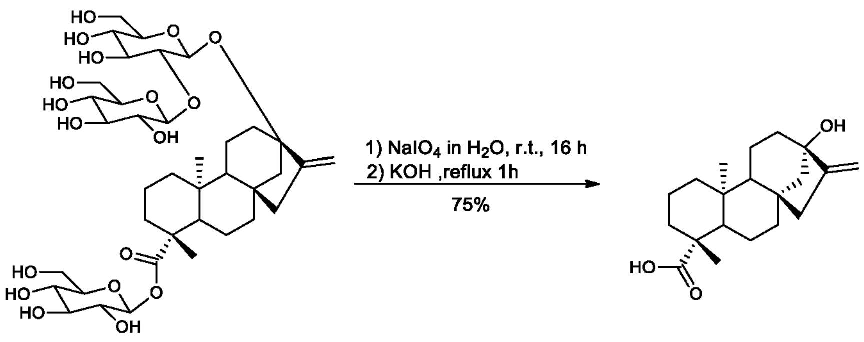


SR1 SR5

**Scheme 9**

**SR5**

White powder; 75 % yield; melting point: 205 °C ± 0.5 °C; [α ]$_D$-45.7°($CDCl_3$). $^1$H NMR ($CDCl_3$, 400 MHz): δ (ppm) = 4.98 (s, 1H, H-17a); 4.81 (s, 1H, H-17b); 2.22-2.07 (m, 4H); 1.95-1.30 (m, 14H); 1.23 (s, 3H, $CH_3$-18); 1.10-0.99 (m, 2H); 0.95 (s, 3H, $CH_3$-20). $^{13}$C NMR ($CDCl_3$, 100 MHz): δ (ppm) = 183.30, 155.85, 103.17, 80.48, 56.99, 53.93, 47.52, 47.03, 43.71, 41.84, 41.35, 40.64, 39.62, 39.45, 37.86, 28.92, 21.92, 20.57, 19.13, 15.55.

ESI-MS (negative) m/z: $[M-H]^-$ calculated for $C_{20}H_{30}O_3$ 317.45; found 317.50.

Synthesis of compound FDA-H (reference example)

A solution consisting of FDA (0.329 mmol, 95 mg) and Pd/C (6.55 mg, 10%) in dry EtOH (16.4 ml) is left under stirring, under a hydrogen atmosphere (10 bar), at room temperature for 24 h. The solution is subsequently filtered, and the solvent is evaporated under pressure, obtaining the FDA-H compound (0.327 mmol, 95 mg) with a quantitative yield. (Murillo, JA et al., 2019).

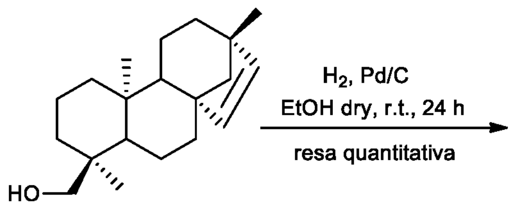


FDA FDA-H

**Scheme 10**

**FDA-H**

White powder (quantitative yield); m.p. 108 °C ± 0.5 °C; [a]$_D$ -4.6°($CHCl_3$). $^1$H NMR ($CDCl_3$, 400 MHz): δ (ppm) = 3.40 (d, 1H, J = 10.8 Hz, H-18a); 3.10 (d, 1H,J = 11.2 Hz, H-18b); 2.03 (m, 1H, H-3eq.); 1.61-1.07(m, 21H); 0.95 (s, 3H, $CH_3$-17); 0.93 (s, 3H, $CH_3$-19); 0.75 (s, 3H, $CH_3$-20); $^{13}$C NMR ($CDCl_3$, 100 MHz): δ (ppm)= 72.45, 57.79, 56.96,49.67, 45.06, 41.01, 40.14, 39.45, 39.41, 37.77, 37.64, 35.39, 33.90, 29.85, 27.32, 20.63, 20.08 , 17.92, 17.90, 15.74.

Synthesis of compound FDO-H

To a solution containing the FDA-H compound (0.258 mmol, 75 mg) in $Et_2O$ (0.192 mmol/ml, 1.34 ml) oxalyl chloride (0.516 mmol, 0.26 ml) is added dropwise at 0 °C. (ratio between starting substrate and reactive 1:2). The solution is left under stirring to reflux for 30 minutes and at room temperature. The reaction is then quenched by slowly adding distilled $H_2O$ until there is no more effervescence. The aqueous solution is extracted with $Et_2O$ and the organic phase thus obtained is washed twice with water and once with brine, and dehydrated on $Na_2SO_4$. The residue is evaporated under pressure and purified through a flash chromatographic column using an eluent mixture $CHCl_3$: $CH_3OH$: HCOOH in a ratio 98: 2: 1%. The FDO-H compound (0.196 mmol; 71 mg) was obtained with a yield of 76%. (Zhang, X. et al., 2016).

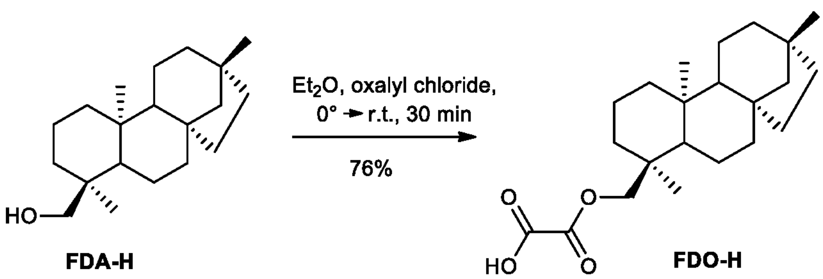


FDA-H FDO-H

**Scheme 11**

**FDO-H**

Light yellow oil (yield 76%); [a]$_D$ -5° ($CHCl_3$); $^1$H NMR ($CDCl_3$, 400 MHz): δ (ppm) = 4.10 (d, 1H, J = 10.8 Hz,

H-18a); 3.90 (d, 1H, J = 10.8 Hz, H-18b); 2.03 (m, 1H, H-3eq.); 0.96 (s, 3H, $CH_3$-17); 1.70-1.07(m, 21H) ; 0.93 (s, 3H, $CH_3$-19); 0.89 (s, 3H, $CH_3$-20). $^{13}C$ NMR ($CDCl_3$, 100 MHz): $\delta$ (ppm) = 158.5, 157.80, 57.67, 56.88, 50.62, 44.98, 40.81, 40.04, 39.44, 39.12, 37.75, 37.71, 36.95, 35.83, 33.84, 29.85, 27.27, 20.57, 20.46, 17.62, 17.59, 15.69. ESI-HRMS (negative) m/z: $[M-H]^-$ calculated for $C_{22}H_{33}O_4$ 361.23843; found 361.23816.

***Biological activity assays***

Bacterial growth inhibition assays

For the evaluation of the antibacterial activity and synergy with colistin of the compounds under examination, a colistin resistant P. *aeruginosa* strain (PA14 $col^R$ 55) with a minimum inhibitory concentration (MIC) of colistin equal to 64 $\mu$g/ml, previously obtained by *in vitro* growth in the presence of increasing concentrations of colistin (Lo Sciuto e Imperi, 2018). This strain over-expresses the *arn* genes responsible for the modification of lipopolysaccharide by the addition of L-amino-arabinose.

The strain was grown in Mueller-Hinton (MH) medium for 8 hours, and inoculated at a cell density of ~$10^6$/ml in MH medium supplemented or not with colistin at a concentration of 16 $\mu$g/ml. One hundred $\mu$l of the bacterial cultures were aliquoted in the wells of a 96-well microtitration plate, containing 100 $\mu$l of MH medium supplemented with decreasing concentrations of the compound to be tested (500, 250, 125, 62.5, 31.25, 16.625, 8.313 or 4.156 $\mu$M) or without compound, so as to bring the bacterial concentration to $5\times10^5$ cells/ml and that of the compounds to 8 $\mu$g/ml for colistin and in the range 0-250 $\mu$M for the compounds under examination, in a final volume of 200 $\mu$l. The microtitration plates were incubated at 37°C without stirring for 24 hours. Bacterial growth was assessed by measuring absorbance at 600 nm ($A_{600}$) in a microplate reader (Victor $2^V$, Perkin-Elmer). Since the compounds are dissolved in DMSO at a concentration of 10 mM, in each assay the bacterial growth is also analyzed in the presence of DMSO at concentrations equivalent to those present in the samples that contain the compounds (0-2.5%) and in the presence or absence of 8 $\mu$g/ml colistin. The 50% or 90% inhibitory concentration ($IC_{50}$ and $IC_{90}$) for each compound was determined as the minimum concentration of the compound capable of causing at least 50% or 90% of bacterial growth inhibition compared to control cultures grown under the same conditions in the presence of an equivalent concentration of DMSO. For each compound three independent experiments were conducted, and the averages of the values obtained in the three experiments are considered to determine the values of $IC_{50}$ e $IC_{90}$.

Checkerboard essays

To further confirm the synergistic activity with colistin of the diterpene compounds under examination, checkerboard essays were carried out, which allow to evaluate the antibacterial activity of the compounds under examination (diterpene derivative and colistin) using different combinations of concentration of the two compounds (Figure 1). For these essays the most promising compounds were selected based on the previously obtained results (BBN149, FDM, FDS, SR4, SR8 and SR10; Tables 2-3). Again, the greatest synergistic activity was found in the compound BBN149, which was able to reduce the minimum inhibitory concentration (MIC) of colistin for the colistin-resistant strain of *P. aeruginosa* by 8 times (64 to 8 $\mu$g/ml) at concentrations of BBN149 $\geq$ 31 $\mu$M, and 4 times (64 to 16 $\mu$g/ml) at concentrations of BBN149 $\geq$ 16 $\mu$M (Figure 1). Similarly, the FDO-H compound reduces the MIC of colistin by 8 times at concentrations $\geq$ 31 $\mu$M while decreasing it by 4 times at concentrations $\geq$ 4 $\mu$M. Compounds FDS, FDM, SR8 and SR10 showed slightly less activity, managing to cause at most a 4-fold reduction in the MIC of colistin (64 to 16 $\mu$g/ml) at minimum concentrations of compound between 16 and 31 $\mu$M. Compound SR4 was the least active, being able to reduce the MIC of colistin by 4 times only at a concentration equal to 125 $\mu$M (Figure 1).

The checkerboard essays were performed in microtiter plates with 96 wells, aliquoting in each column of wells 50 $\mu$l of MH medium containing decreasing concentrations of colistin (dilutions in reason of 2 from 384 to 1.5 $\mu$g/ml) or without colistin, and in each row of wells further 50 $\mu$l of MH medium containing decreasing concentrations of compound of interest (dilutions in reason 2 from 375 to 11.7 $\mu$g/ml) or without compound. Finally, 50 $\mu$l of the inoculum of the PA14 $col^R$ 5 strain were added to each well at a concentration of ~$1.5\times10^6$ cells/ml in MH medium, so as to reach a final volume of 150 $\mu$l, a bacteria concentration equal to $0.5\times10^5$ cells/ml, colistin concentrations in the range 0-128 $\mu$g/ml and concentrations of compounds of interest in the range 0-125 $\mu$M. Plates were incubated at 37°C without shaking for 24 hours, and bacterial growth was assessed by measuring the $A_{600}$ in a microplate reader (Victor $2^V$, Perkin-Elmer). Three independent experiments were conducted for each compound, and the averages of the values obtained in the three experiments were considered to determine the $IC_{90}$ values.

Minimum inhibitory concentration (MIC) essays: Activity spectrum and specificity of the compound BBN149

To evaluate the spectrum of activity and the specificity of the compound BBN149, which was found to be the

most effective compound in enhancing the activity of colistin (Tables 1-3 and Figure 1), MIC essays were performed using different strains of *P. aeruginosa,* both resistant and sensitive to colistin, and colistin-resistant clinical strains of another Gram-negative bacterium, *Klebsiella pneumoniae.* All the colistin-resistant strains used depend on the aminoarabinosylation of lipid A as a mechanism of resistance to colistin (Lo Sciuto e Imperi, 2018; Esposito et al., 2018). As reported in Table 5, the compound BBN149 was able to reduce the MIC of colistin, by a value between 4 and 16 times, in all the colistin-resistant strains analyzed, both of *P. aeruginosa* and of *K. pneumoniae.* Furthermore, the compound showed no relevant activity on colistin-sensitive strains (Table 5). Overall, these data demonstrate that the BBN149 compound is able to specifically interfere with the mechanism of resistance to colistin in various Gram-negative bacteria. MIC essays were performed in 96-well microtitration plates, using previously characterized P. aeruginosa and K. pneumoniae strains (Lo Sciuto e Imperi, 2018; Esposito et al., 2018). In each column of wells were aliquoted 100 μl of MH medium containing decreasing concentrations of colistin (dilutions in reason 2 from 256 to 0.5 μg/ml) or without colistin, and in each row of wells further 100 μl of MH medium containing ~ 1 × $10_6$ cells/ml of each bacterial strain of interest and BBN149 or DMSO as a control at a concentration of 120 μM or 1.2% respectively, so as to reach a final volume of 200 μl, an equal concentration of bacteria at 0.5 × $10_5$ cells/ml, colistin concentrations in the range 0-128 μg/ml and concentrations of BBN140 or DMSO equal to 60 μM or 0.6% respectively. The plates were incubated at 37°C without stirring for 24 hours, and the MIC was visually evaluated as the minimum concentration of colistin capable of causing absence of turbidity in the well. At least three independent experiments were conducted for each strain.

Table 5. MIC of colistin for different strains of *P. aeruginosa* and *K. pneumoniae* in the presence of 60 μM BBN149 or 0.6% DMSO as a control.

| Specie | Strain | MIC (μg/ml) | |
|---|---|---|---|
| | | BBN149 | DMSO |
| *P. aeruginosa* | PA14 $col^R$ 5 | 8 | 64 |
| | PA14 | 1 | 0.5 |
| | KK1 $col^R$ 1 | 8 | 128 |
| | KK1 | 1 | 0.5 |
| | KK27 $col^R$ 6 | 4 | 64 |
| | KK27 | 1 | 0.5 |
| | TR1 $col^R$ 6 | 4 | 16 |
| | TR1 | 1 | 0.5 |
| *K. pneumoniae* | KP-Mo-3 | 16 | 128 |
| | KP-Mo-5 | 8 | 128 |
| | KP-Mo-6 | 4 | 32 |
| | KP-Mo-11 | 8 | 64 |
| | KP-Mo-16 | 8 | 64 |

*Cell viability test*

The cytotoxic potential of the compounds was evaluated on cell cultures *in vitro.* For these essays, the most promising compounds were selected based on their antibacterial activity in synergy with colistin. In particular, for the compounds BBN149, FDM, FDS, SR4, SR8 and SR10, cytotoxic activity was determined on human epithelial cells of bronchial origin, 16HBE (Cozens et al, 1994). All compounds were tested in the concentration range of 1.95 μM to 250 μM with an exposure of 18 hours. The results are reported in the table as viability (%) compared to untreated cells (Table 4). Cell viability of control samples treated only with the solvent (DMSO) was equal to 97.11% (±7.65) regardless of the concentration used, in the range between 2.5% and 0.02% (1/2 serial dilutions). Although almost all the compounds show a reduction in cell viability from 15% to 35% at the highest concentrations (250 μM), at concentrations active against *P. aeruginosa,* cell viability is reduced by a maximum of about 10%. Compounds SR4 and SR10 show a total reduction of cell viability, exclusively at the highest concentration (250 μM). This result can be explained by the reduced solubility of the compound in aqueous media and therefore by the possible precipitation in the cell culture medium.

To evaluate the cytotoxic potential of the compounds under examination, the MTT (3-(4,5-dimethylthiazol-2-yl)-2,5-diphenyltetrazolium) reduction test, commonly used to monitor cell viability was used following the exposure to potentially cytotoxic agents. The essay is based on the reduction of MTT to formazan, a compound that assumes a blue colour which can be evaluated by spectrophotometric reading at a wavelength of 570 nm. For the essay we used the 16HBE cell line. 16HBE cells are derived from human bronchi and their production is reported in Cozens et al (1994). The essay was performed as follows: $3 \times 10^4$ cells per well were seeded in 96-well multi-well plates and incubated (37°C, 5% $CO_2$) to allow adhesion to the bottom of the plate (8-12 hr); the compounds dissolved in DMSO at a concentration of 10 mM were added to a final concentration of 250 μM and diluted 1/2 up to 1.95 μM in the corresponding wells; similarly control samples were treated with 2.5% DMSO (equal to the amount of DMSO present in the samples treated with the compounds at 250 μM) and serially diluted 1/2 up to 0.019%; the cells were then incubated for 18 hours (37°C, 5% $CO_2$); MTT 0.5 mg/ml was added to each well of the plate and the plate was incubated again for 3 hours (37°C, 5% $CO_2$); after incubation, the supernatant was removed and the formazan was solubilized in DMSO; the quantity of formazan produced was determined by spectrophotometric reading at a wavelength of 570 nm. Each test was performed in duplicate and three independent replicas were made.

Cell viability was calculated as follows:

$$C_{OD570} = OD_{570} \text{ of sample - } OD_{570} \text{ of wells without cells (white)}$$

$$\text{Reduction (\%)} = (C_{OD570}NT - C_{OD570}T) / C_{OD570}NT \times 100$$

$$\text{Viability} = 100 - \text{reduction (\%)}$$

where: NT indicates the samples of cells non-threated with the compounds; T the samples of cells treated with the compounds at the different concentrations.

The mean cell viability values were calculated using the Excel mean and standard deviation functions.

Table 4. Average percentage of cell viability (± standard deviation)

| μM | FDM | BBN149 | FDS | SR4 | SR8 | SR10 | FDO-H | DMSO |
|---|---|---|---|---|---|---|---|---|
| 250 | 85.01 ± 2.40 | 65.51 ± 4.05 | 86.12 ± 3.18 | -1.11 ± 2.02 | 80.16 ± 2.01 | 1.47±2.29 | 76.51±12.64 | 72.15±19.63 |
| 125 | 100.48±12.78 | 71.99 ± 5.18 | 105.88 ± 1.31 | 106.23 ± 5.52 | 78.02 ± 7.46 | 92.07±5.47 | 98.42±16.71 | 76.74±6.62 |
| 62.5 | 106.63 ± 2.87 | 76.33 ± 2.65 | 102.15 ± 3.15 | 99.27 ± 2.43 | 85.68 ± 6.99 | 99.80±4.30 | 97.14±12.19 | 85.58±9.56 |
| **31.25** | **113.17 ± 8.95** | **86.07 ± 5.30** | **102.95 ± 6.74** | **101.69 ± 4.39** | **90.55 ± 4.17** | **99.62±9.62** | 93.65±11.27 | 87.92±5.29 |
| **15.63** | **107.42 ± 0.01** | **90.80 ± 6.31** | **99.77 ± 6.58** | **97.27 ± 5.61** | **92.64 ± 6.57** | **97.98±1.66** | 94.06±3.88 | 92.29±5.70 |
| 7.81 | 97.93 ± 2.17 | 86.13 ± 2.37 | 93.53 ± 2.01 | 92.44 ± 2.11 | 90.33 ± 4.26 | 101.00±7.12 | 100.25±23.84 | 90.21±3.93 |
| 3.91 | 96.70 ± 1.60 | 90.17 ± 7.35 | 95.34 ± 8.28 | 92.31 ± 3.26 | 97.84 ± 10.19 | 104.95±6.36 | 94.57±12.67 | 97.03±9.44 |
| 1.95 | 102.92 ± 7.31 | 94.15 ± 6.34 | 98.93 ± 5.38 | 96.35 ± 9.42 | 100.17±14.21 | 105.21±6.73 | 106.31±17.23 | 105.83±6.65 |

## Claims

1. A compound selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**) and ent-beyeran-18-O-oxalic acid (**FDO**-**H**) and pharmaceutically acceptable salts thereof for use as an adjuvant in antibiotic therapy in the treatment of polymyxin-resistant bacterial infections.

2. Compound for use according to claim 1, wherein the polymyxins-resistant bacterial infections are bacterial infections resistant to colistin (polymyxin E) or polymyxin B.

3. Compound selected from: glucopyranosyl β-D ester of ent-beyeran-19-oic acid (**SR4**), ent-beyer-15-en-18-O-malonic acid (**FDM**) and ent-beyeran-18-O-oxalic acid (**FDO**-**H**).

4. Compound according to claim 3, for use as a medicament.

5. Compound for use according to any one of claims 1 or 2, wherein the bacterial infections are caused by a Gram-negative bacterium selected from *Pseudomonas aeruginosa, Escherichia coli, Klebsiella pneumoniae, Klebsiella oxytoca, Enterobacter spp, Citrobacter freundii, Salmonella typhimurium, Burkholderia cenocepacia, Yersinia pestis, Yersinia enterocolitica, Proteus mirabilis* and *Salmonella enterica serovar Typhimurium.*

6. Compound for use according to any one of claims 1 to 2 or 5, wherein the infection is an acute or chronic pulmonary, extrapulmonary localized or systemic infection.

7. Compound for use according to any one of claims 1 to 2 or 5 to 6, wherein the compound is for use as an adjuvant in an antibiotic therapy with polymyxins, preferably colistin (polymyxin E) or polymyxin B.

8. Combination of one or more of the compounds selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**), ent-beyeran-18-O-oxalic acid (**FDO**-**H**) and pharmaceutically acceptable salts thereof with at least another active principle, wherein the active principle is an antibacterial agent and/or an antibiotic, preferably wherein the antibiotic belongs to the class of polymyxins, more preferably wherein the antibiotic is colistin (polymyxin E) or polymyxin B.

9. Combination according to claim 8, wherein the weight ratio between the first compound and the antibacterial agent and/or the antibiotic is between 1:1-1:20.

10. Composition comprising at least a compound selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**), ent-beyeran-18-O-oxalic acid **(FDO-H)** and pharmaceutically acceptable salts thereof or the combination according to anyone of claims 8 or 9 and at least a pharmaceutically acceptable excipient or carrier.

11. Composition according to claim 10, in liquid, solid or semisolid form, preferably in form of solution, suspension, cream or ointment.

12. Bandage, gauze, patch, cotton wool, spray, prostheses or probes comprising a compound selected from ent-beyer-15-en-18-O-oxalic acid **(BBN149);** ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**), ent-beyeran-18-O-oxalic acid (**FDO**-**H**) and pharmaceutically acceptable salts thereof, the combination according to any one of claims 8 to 9, or the composition according to any one of claims 10 to 11.

13. *In vitro* use of a compound selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**), ent-beyeran-18-O-oxalic acid (**FDO**-**H**) and pharmaceutically acceptable salts thereof to sensitize a bacterium to an antibacterial agent or an antibiotic, wherein the antibiotic preferably belongs to the class of polymyxins and more preferably is colistin (polymyxin E) or polymyxin B.

14. An *in vitro* method for sensitizing a bacterium to an antibacterial agent or an antibiotic comprising the exposure of said bacterium to one or more compounds selected from ent-beyer-15-en-18-O-oxalic acid (**BBN149**); ent-beyer-15-en-18-O-malonic acid (**FDM**); ent-beyer-15-en-18-O-succinic acid (**FDS**); glucopyranosyl β-D ester of ent-beyeran-19-oic acid (**SR4**); ent-beyeran-19-O-oxalic acid (**SR8**); ent-beyeran-19-oic acid (**SR10**), ent-beyeran-18-O-oxalic acid (**FDO-H**) and pharmaceutically acceptable salts thereof or to the combination according to any one of claims 9 to 10.

## Patentansprüche

1. Verbindung, ausgewählt aus Ent-Beyer-15-en-18-O-Oxalsäure (**BBN149**); Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Säure (**SR10**) und Ent-Beyeran-18-O-Oxalsäure (**FDO-H**) und deren pharmazeutisch verträglichen Salzen zur Verwendung als Adjuvans in der Antibiotikatherapie zur Behandlung von Infektionen mit polymyxinresistenten Bakterien.

2. Verbindung zur Verwendung nach Anspruch 1, wobei die Infektionen mit polymyxinresistenten Bakterien Infektionen mit Bakterien sind, die gegen Colistin (Polymyxin E) oder Polymyxin B resistent sind.

3. Verbindung, ausgewählt aus: Glucopyranosyl-β-D-Ester von Ent-Beyeran-19-Säure (**SR4**), Ent-Beyer-15-en-18-O-Malonsäure (**FDM**) und Ent-Beyeran-18-O-Oxalsäure (**FDO-H**).

4. Verbindung nach Anspruch 3 zur Verwendung als Medikament.

5. Verbindung zur Verwendung nach einem der Ansprüche 1 oder 2, wobei die bakteriellen Infektionen durch ein gramnegatives Bakterium verursacht werden, ausgewählt aus *Pseudomonas aeruginosa, Escherichia coli, Klebsiella pneumoniae, Klebsiella oxytoca, Enterobacter spp., Citrobacter freundii, Salmonella typhimurium, Burkholderia cenocepacia, Yersinia pestis, Yersinia enterocolitica, Proteus mirabilis* und *Salmonella enterica Serovar Typhimurium.*

6. Verbindung zur Verwendung nach einem der Ansprüche 1 bis 2 oder 5, wobei die Infektion eine akute oder chronische pulmonale, extrapulmonale lokalisierte oder systemische Infektion ist.

7. Verbindung zur Verwendung nach einem der Ansprüche 1 bis 2 oder 5 bis 6, wobei die Verbindung zur Verwendung als Adjuvans in einer Antibiotikatherapie mit Polymyxinen, vorzugsweise Colistin (Polymyxin E) oder Polymyxin B, dient.

8. Kombination aus einer oder mehreren der Verbindungen, ausgewählt aus Ent-Beyer-15-en-18-O-Oxalsäure (**BBN149**); Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Oxalsäure (**SR10**), Ent-Beyeran-18-O-Oxalsäure (**FDO-H**) und pharmazeutisch verträglichen Salzen davon mit mindestens einem anderen Wirkprinzip, wobei das Wirkprinzip ein antibakterielles Mittel und/oder ein Antibiotikum ist, wobei das Antibiotikum vorzugsweise zur Klasse der Polymyxine gehört, und wobei das Antibiotikum bevorzugter Colistin (Polymyxin E) oder Polymyxin B ist.

9. Kombination nach Anspruch 8, wobei das Gewichtsverhältnis zwischen der ersten Verbindung und dem antibakteriellen Mittel und/oder dem Antibiotikum zwischen 1:1 und 1:20 liegt.

10. Zusammensetzung, umfassend mindestens eine Verbindung, ausgewählt aus Ent-Beyer-15-en-18-O-Oxalsäure (**BBN149**); Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Säure (**SR10**), Ent-Beyeran-18-O-Oxalsäure (**FDO-H**) und pharmazeutisch verträglichen Salzen davon oder die Kombination nach einem der Ansprüche 8 oder 9 und mindestens einen pharmazeutisch verträglichen Hilfs- oder Trägerstoff.

11. Zusammensetzung nach Anspruch 10 in flüssiger, fester oder halbfester Form, vorzugsweise in Form einer Lösung, Suspension, Creme oder Salbe.

12. Verband, Gaze, Pflaster, Watte, Spray, Prothesen oder Sonden, umfassend eine Verbindung, ausgewählt aus Ent-Beyer-15-en-18-O-Oxalsäure (**BBN149**); Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Oxalsäure (**SR10**), Ent-Beyeran-18-O-Oxalsäure **(FDO-H)** und pharmazeutisch verträglichen Salzen davon, die Kombination nach einem der Ansprüche 8 bis 9 oder die Zusammensetzung nach einem der Ansprüche 10 bis 11.

13. *In-vitro*-Verwendung einer Verbindung, ausgewählt aus Ent-Beyer-15-en-18-O-Oxalsäure (**BBN149**); Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Oxalsäure (**SR10**), Ent-Beyeran-18-O-Oxalsäure (**FDO-H**) und pharmazeutisch verträglichen Salzen davon zur Sensibilisierung eines Bakteriums für ein antibakterielles Mittel oder ein Antibiotikum, wobei das Antibiotikum vorzugsweise zur Klasse der Polymyxine gehört und bevorzugter Colistin (Polymyxin E) oder Polymyxin B ist.

14. *In-vitro*-Verfahren zum Sensibilisieren eines Bakteriums gegenüber einem antibakteriellen Mittel oder einem Antibiotikum, umfassend die Aussetzung des Bakteriums gegenüber einer oder mehreren Verbindungen, ausgewählt aus Ent-Beyer-15-en-18-O-Oxalsäure **(BBN149);** Ent-Beyer-15-en-18-O-Malonsäure (**FDM**); Ent-Beyer-15-en-18-O-Bernsteinsäure (**FDS**); Glucopyranosyl-β-D-Ester von Ent-Beyeran-19-Säure (**SR4**); Ent-Beyeran-19-O-Oxalsäure (**SR8**); Ent-Beyeran-19-Säure (**SR10**), Ent-Beyeran-18-O-Oxalsäure (**FDO-H**) und pharmazeutisch verträglichen Salzen davon oder auf die Kombination nach einem der Ansprüche 9 bis 10.

**Revendications**

1. Composé choisi parmi l'acide ent-beyer-15-en-18-O-oxalique **(BBN149)** ; l'acide ent-beyer-15-en-18-0-malonique **(FDM)** ; l'acide ent-beyer-15-en-18-0-succinique **(FDS)** ; l'ester glucopyranosyl β-D de l'acide ent-beyeran-19-oïque **(SR4)** ; l'acide ent-beyeran-19-O-oxalique (**SR8**) ; l'acide ent-beyeran-19-oïque (**SR10**) et l'acide ent-beyeran-18-O-oxalique **(FDO-H)** et leurs sels pharmaceutiquement acceptables pour une utilisation comme adjuvant d'une antibiothérapie dans le traitement des infections bactériennes résistantes à la polymyxine.

2. Composé utilisé selon la revendication 1, dans lequel les infections bactériennes résistantes aux polymyxines sont des infections bactériennes résistantes à la colistine (polymyxine E) ou à la polymyxine B.

3. Composé choisi parmi : l'ester glucopyranosyl β-D de l'acide ent-beyeran-19-oïque (**SR4**), l'acide ent-beyer-15-en-18-0-malonique (**FDM**) et l'acide ent-beyeran-18-O-oxalique (**FDO-H**).

4. Composé selon la revendication 3, destiné à être utilisé comme médicament.

5. Composé destiné à être utilisé selon l'une quelconque des revendications 1 ou 2, dans lequel les infections bactériennes sont causées par une bactérie Gram négative choisie parmi *Pseudomonas aeruginosa, Escherichia coli, Klebsiella pneumoniae,* Klebsiella *oxytoca, Enterobacter spp, Citrobacter freundii, Salmonella typhimurium, Burkholderia cenocepacia, Yersinia pestis, Yersinia enterocolitica, Proteus mirabilis* et *Salmonella enterica serovar Typhimurium.*

6. Composé destiné à être utilisé selon l'une quelconque des revendications 1 à 2 ou 5, dans lequel l'infection est une infection pulmonaire aiguë ou chronique, extrapulmonaire localisée ou systémique.

7. Composé destiné à être utilisé selon l'une des revendications 1 à 2 ou 5 à 6, dans lequel le composé est destiné à être utilisé comme adjuvant dans une antibiothérapie à base de polymyxines, de préférence la colistine (polymyxine E) ou la polymyxine B.

8. Combinaison d'un ou plusieurs composés choisis parmi l'acide ent-beyer-15-en-18-O-oxalique **(BBN149)** ; l'acide ent-beyer-15-en-18-0-malonique **(FDM)** ; l'acide ent-beyer-15-en-18-O-succinique **(FDS)** ; l'ester glucopyranosyl β-D de l'acide ent-beyeran-19-oïque **(SR4)** ; l'acide ent-beyeran-19-O-oxalique **(SR8**) ; l'acide ent-beyeran-19-oïque **(SR10**), l'acide ent-beyeran-18-O-oxalique **(FDO-H)** et leurs sels pharmaceutiquement acceptables avec au moins un autre principe actif, dans lequel le principe actif est un agent antibactérien et/ou un antibiotique, de préférence dans lequel l'antibiotique appartient à la classe des polymyxines, plus préférablement dans lequel l'antibiotique est la colistine (polymyxine E) ou la polymyxine B.

**9.** Combinaison selon la revendication 8, dans laquelle le rapport en poids entre le premier composé et l'agent antibactérien et/ou l'antibiotique est compris entre 1:1 - 1:20.

**10.** Composition comprenant au moins un composé choisi parmi l'acide ent-beyer-15-en-18-O-oxalique **(BBN149**) ; l'acide ent-beyer-15-en-18-0-malonique **(FDM)** ; l'acide ent-beyer-15-en-18-O-succinique **(FDS)** ; l'ester glucopyranosyl β-D de l'acide ent-beyeran-19-oïque **(SR4)** ; l'acide ent-beyeran-19-O-oxalique **(SR8**) ; l'acide ent-beyeran-19-oïque (**SR10**), l'acide ent-beyeran-18-O-oxalique (**FDO-H**) et leurs sels pharmaceutiquement acceptables ou la combinaison selon l'une quelconque des revendications 8 ou 9 et au moins un excipient ou un support pharmaceutiquement acceptable.

**11.** Composition selon la revendication 10, sous forme liquide, solide ou semi-solide, de préférence sous forme de solution, de suspension, de crème ou de pommade.

**12.** Bandage, gaze, timbre cutané, coton, spray, prothèses ou sondes comprenant un composé choisi parmi l'acide ent-beyer-15-en-18-O-oxalique **(BBN149)** ; l'acide ent-beyer-15-en-18-O-malonique **(FDM)** ; l'acide ent-beyer-15-en-18-O-succinique **(FDS)** ; l'ester glucopyranosyl β-D de l'acide ent-beyeran-19-oïque **(SR4)** ; l'acide ent-beyeran-19-O-oxalique (**SR8**) ; l'acide ent-beyeran-19-oïque (**SR10**), l'acide ent-beyeran-18-O-oxalique (**FDO-H**) et leurs sels pharmaceutiquement acceptables, la combinaison selon l'une quelconque des revendications 8 à 9, ou la composition selon l'une quelconque des revendications 10 à 11.

**13.** Utilisation *in vitro* d'un composé choisi parmi l'acide ent-beyer-15-en-18-0-oxalique **(BBN149**) ; l'acide ent-beyer-15-en-18-O-malonique **(FDM)** ; l'acide ent-beyer-15-en-18-0-succinique **(FDS)** ; l'ester glucopyranosyl β-D de l'acide ent-beyeran-19-oïque **(SR4)** ; l'acide ent-beyeran-19-O-oxalique **(SR8**) ; l'acide ent-beyeran-19-oïque (**SR10**), l'acide ent-beyeran-18-O-oxalique **(FDO-H**) et leurs sels pharmaceutiquement acceptables pour sensibiliser une bactérie à un agent antibactérien ou à un antibiotique, l'antibiotique appartenant de préférence à la classe des polymyxines et plus préférablement à la colistine (polymyxine E) ou à la polymyxine B.

**14.** Procédé *in vitro* de sensibilisation d'une bactérie à un agent antibactérien ou à un antibiotique comprenant l'exposition de ladite bactérie à un ou plusieurs composés choisis parmi l'acide ent-beyer-15-en-18-O-oxalique (**BBN149**) ; l'acide ent-beyer-15-en-18-O-malonique **(FDM)** ; l'acide ent-beyer-15-en-18-0-succinique **(FDS)** ; l'ester glucopyranosyl β-D de l'acide ent-beyeran-19-oïque **(SR4)** ; l'acide ent-beyeran-19-O-oxalique **(SR8**) ; l'acide ent-beyeran-19-oïque (**SR10**), l'acide ent-beyeran-18-O-oxalique (**FDO-H**) et leurs sels pharmaceutiquement acceptables ou à la combinaison selon l'une quelconque des revendications 9 à 10.

FIG. 1

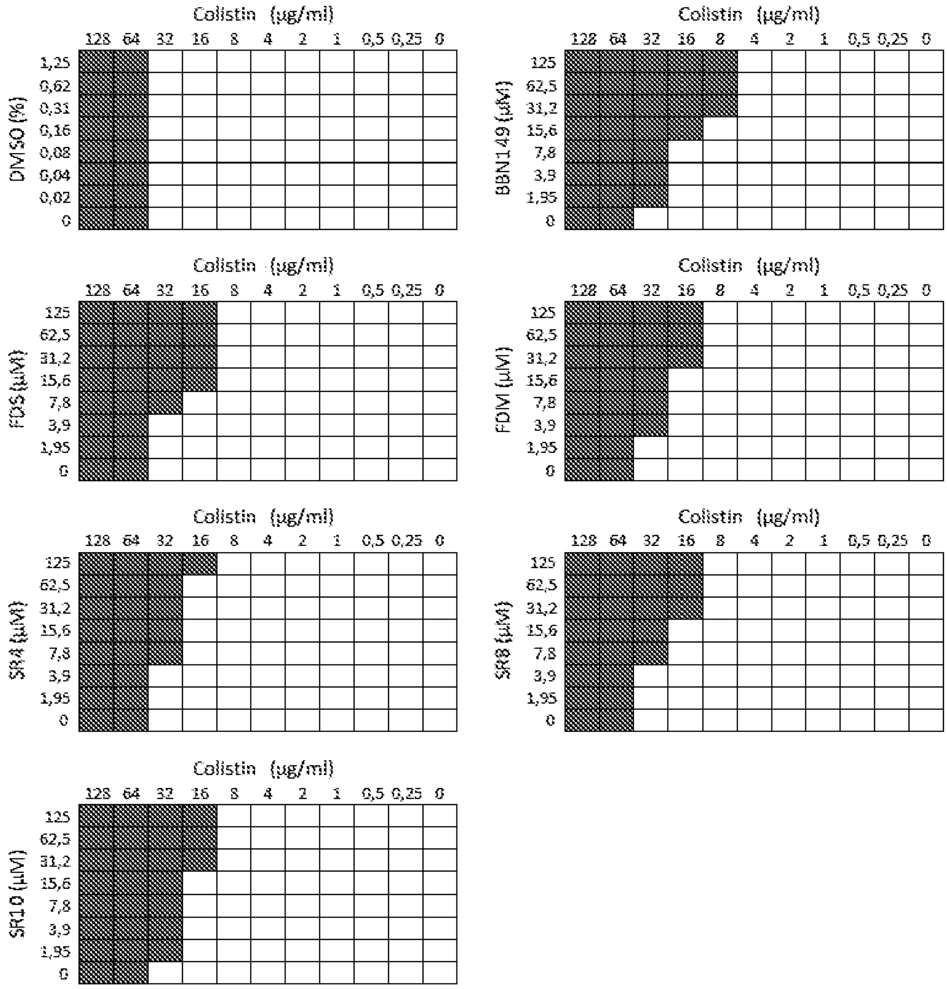
Colistin (µg/ml)
128 64 32 16 8 4 2 1 0,5 0,25 0
DMSO (%)
1,25 0,62 0,31 0,16 0,08 0,04 0,02 0
BBN149 (µM)
125 62,5 31,2 15,6 7,8 3,9 1,95 0
FDS (µM)
FDM (µM)
SR4 (µM)
SR8 (µM)
SR10 (µM)

FIG. 2

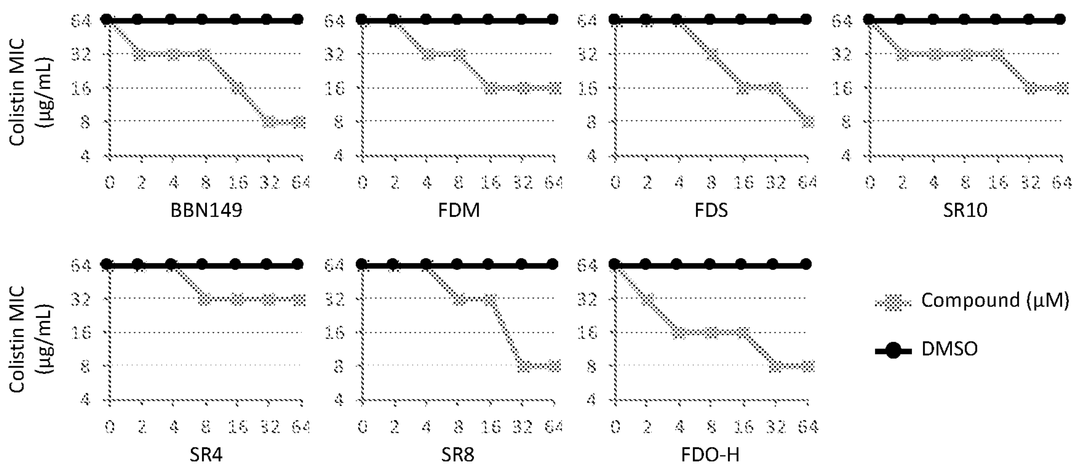
Colistin MIC (µg/mL)
64 32 16 8 4
0 2 4 8 16 32 64
BBN149
FDM
FDS
SR10
SR4
SR8
FDO-H
Compound (µM)
DMSO

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017083859 A


### Non-patent literature cited in the description


- *Protein Expression and Purification,* March 2006, vol. 46 (1), 33-39
- **LYNN E. BRETSCHER et al.** *Purification and characterization of the L-Ara4N transferase protein ArnT from Salmonella typhimurium, https://doi.org/10.1016/j.pep.2005.08.028*
- **CHARLOTTE OLAGNON et al.** Synthetic Phosphodiester-Linked 4-Amino-4-deoxy-l-arabinose Derivatives Demonstrate that ArnT is an Inverting Aminoarabinosyl Transferase. *ChemBioChem,* 2019, vol. 20, 2936-2948
- **KLINE T et al.** Synthesis of and evaluation of lipid A modification by 4-substituted 4-deoxy arabinose analogues as potential inhibitors of bacterial polymyxin resistance. *Bioorg Med Chem Lett.,* 2008
- **CAI C. et al.** Two new kaurane-type diterpenoids from Wedelia chinensis (Osbeck.) Merr. *Nat. Prod. Res.,* 2017, vol. 31 (21), 25-31
- **DREWES S. E. et al.** Antimicrobial monomeric and dimeric diterpenes from the leaves of Helichrysum tenax var tenax. *Phytochem.,* 2006, vol. 67 (7), 716
- **MCCHESNEY J.D. et al.** Antimicrobial diterpenes of Croton sondenarius. II ent-Beyer-15-en-18-oic Acid. *Pharm. Res.,* 1991, vol. 8 (10), 1243
- **LO SCIUTO A ; MARTORANA AM ; FERNÁNDEZ-PIÑAR R ; MANCONE C ; POLISSI A ; IMPERI F.** Pseudomonas aeruginosa LptE is crucial for LptD assembly, cell envelope integrity, antibiotic resistance and virulence. *Virulence,* 2018, vol. 9 (1), 1718-1733
- Breakpoint Tables for Interpretation of MICs and Zone Diameters. *European Committee on Antimicrobial Susceptibility Testing (EUCAST),* 2018, vol. 8
- M07-A10: Methods for Dilution Antimicrobial Susceptibility Tests for Bacteria That Grow Aerobically; Approved Standard. CLSI, Clinical and Laboratory Standards Institute. 2015, vol. 35
- **JEANNOT K ; BOLARD A ; PLÉSIAT P.** Resistance to polymyxins in Gram-negative organisms. *Int J Antimicrob Agents.,* 2017, vol. 49 (5), 526-535
- **JANG HOON K. et al.** *Journal of Enzyme Inhibition and Medicinal Chemistry,* 2017, vol. 32, 78-83